# EUROPÄISCHE PATENTSCHRIFT

(11) **EP 4 178 449 B1**
(45) Veröffentlichungstag und Bekanntmachung des Hinweises auf die Patenterteilung: **25.11.2026**
(21) Anmeldenummer: 21749115.8
(22) Anmeldetag: 12.07.2021
(51) Int. Cl.: A61B 6/04, A61G 7/053

(54) **RADIOLOGIEHALTEEINRICHTUNG FÜR EIN RADIOLOGIEGERÄT**
RADIOLOGY HOLDING UNIT FOR A RADIOLOGY DEVICE
UNITÉ DE MAINTIEN UTILISÉE EN RADIOLOGIE POUR UN DISPOSITIF DE RADIOLOGIE

(30) Priorität: 13.07.2020 DE 102020118448
(43) Veröffentlichungstag der Anmeldung: 17.05.2023
(73) Patentinhaber: febromed GmbH & Co. KG, 59302 Oelde (DE)
(72) Erfinder: BRORMANN, Hubert, 59302 Oelde (DE)
(74) Vertreter: BSB Patentanwälte Schütte & Engelen Partnerschaft mbB
(86) Internationale Anmeldenummer: PCT/EP2021/069364
(87) Internationale Veröffentlichungsnummer: WO 2022/013162

(56) Entgegenhaltungen:
- WO-A1-2021/009138
- DE-U1- 202016 106 093

## Beschreibung

Die vorliegende Erfindung betrifft eine Radiologiehalteeinrichtung insbesondere für ein Radiologiegerät sowie eine Radiologieanlage mit einer solchen Radiologiehalteeinrichtung und einem Radiologiegerät wie insbesondere einem Computertomographen (CT), einem Magnetresonanztomographen (MRT), einem Röntgengerät, einem Strahlentherapiegerät oder dergleichen.

Computertomographen oder Magnetresonanztomographen weisen oft eine Art Röhre auf, durch die der Patient bei der Anfertigung der nötigen Aufnahmen durchgeschoben bzw. durchgefahren wird. Verallgemeinert werden im Folgenden Magnetoresonanztomographen oder Computertomographen oder Röntgengeräte oder dergleichen mehr mit dem Begriff Röntgengerät bezeichnet.

Soll ein bettlägeriger Patient mit einem solchen Röntgengerät geröntgt werden bzw. ein MRT oder ein CT angefertigt werden, so wird ein bettlägeriger Patient in einem Bett liegend oder in einem Rollstuhl sitzend in den mit dem Röntgengerät ausgerüsteten Raum hineingeschoben und neben der Liege bzw. dem Arbeitstisch positioniert. Anschließend muss der Patient aus einem Bett oder einem Rollstuhl oder einer Liege auf den Arbeitstisch umgelagert werden, bevor der MRT- oder CT-Vorgang gestartet werden kann. Diese Tätigkeiten des Umbettens und korrekten Positionierens erfordern viel Kraft und Zeit.

Aus der DE 20 2016 106 093 U1 und aus der unveröffentlichten bzw. nachveröffentlichten DE 10 2019 119 025.8 (DE 10 2019 119 025 A1) sind Radiologiehalteeinrichtungen bekannt geworden, mit der solche Tätigkeiten unterstützt werden können. An einem verschwenkbaren Tragarm ist ein Haltegriff montiert, an dem ein Patient sich festhalten kann. Der Tragarm ist verschwenkbar und ist winkelmäßig fixierbar. Durch Zug an einem Seil, an deren Ende eine Griffkugel befestigt ist, kann ein Bolzen aus einem Lochkreis entriegelt werden, sodass der Tragarm durch die Betätigung entriegelt und dann verschwenkt werden kann.

Bei der DE 10 2019 119 025.8 kann das Zugseil auch durch Führungshülsen horizontal entlang des Tragarms radial nach weiter außen geführt werden, um nicht nur zentral, sondern auch radial weiter außen eine Betätigung zu ermöglichen. Das ist vorteilhaft und vereinfacht die Bedienung. Nachteilig ist dabei aber, dass das Seil durchhängt. Das Seil muss erst straffgezogen werden, bevor der Bolzen den Tragarm zur Drehung freigibt. Abhängig davon, wo an dem Seil gezogen wird, kann das Seil auch (rückwärts) aus der Führung gezogen werden, ohne dass sich der Entriegelungsbolzen bewegt. Der Betätigungsweg, um welchen das durchhängende Seil zur Entriegelung des Bolzens gezogen werden muss, ist abhängig von der Position, an welcher gezogen wird. Außerdem kann sich durch das Ziehen an dem durchhängenden Seil, die Position einer benachbarten, verbundenen und weiter von der Schwenkachse des Tragarms entfernten Griffkugel so verändern, dass diese selbst nicht mehr erreichbar ist. Ein Zugweg zur Entriegelung des Bolzens ist nicht definiert und für einen Benutzer vor allem bei mehrfacher Betätigung unvorhersehbar. Es gibt daher ein Risiko, dass der Bolzen beim Umlagern von Personen nicht vorhersehbar entriegelt werden kann.

Es ist deshalb die Aufgabe der vorliegenden Erfindung, eine Radiologiehalteeinrichtung zur Verfügung zu stellen, mit welcher die Bedienung von Radiologiehalteeinrichtungen erleichtert wird. Vorzugsweise soll eine zuverlässige Betätigung ermöglicht werden. Insbesondere soll auch die Hygiene verbessert werden.

Diese Aufgabe wird durch eine Radiologiehalteeinrichtung mit den Merkmalen des Anspruchs 1 und durch eine Radiologieanlage mit den Merkmalen des Anspruchs 15 gelöst. Bevorzugte Weiterbildungen der Erfindung sind Gegenstand der Unteransprüche. Weitere Vorteile und Merkmale der vorliegenden Erfindung ergeben sich aus der allgemeinen Beschreibung und der Beschreibung des Ausführungsbeispiels.

Eine erfindungsgemäße Radiologiehalteeinrichtung ist insbesondere für wenigstens ein Radiologiegerät und vorzugsweise für wenigstens ein Röntgengerät, ein MRT-Gerät oder ein CT-Gerät oder ein Strahlentherapiegerät oder ein ähnliches oder vergleichbares Gerät vorgesehen. Die Radiologiehalteeinrichtung umfasst eine Befestigungseinrichtung und wenigstens einen daran schwenkbar aufgenommenen Tragarm. Der Tragarm umfasst wenigstens ein bzw. einen Achsteil und wenigstens ein bzw. einen Tragteil. Das Achsteil des (bzw. jedes) Tragarms ist schwenkbar an der Befestigungseinrichtung aufgenommen und insbesondere daran gelagert. Die Radiologiehalteeinrichtung weist wenigstens eine Feststelleinrichtung auf, um den Tragarm in wenigstens einer Schwenkposition bzw. Winkelposition, insbesondere einer von mehreren Arbeitspositionen, festzustellen. Die Winkelposition, in der der Tragarm feststellbar ist, ist eine Feststellposition.

Es ist wenigstens ein Betätigungsmechanismus mit wenigstens einem Übertragungselement und wenigstens einem Betätigungselement vorhanden, um die Feststelleinrichtung zu betätigen und insbesondere zu lösen und/oder festzustellen. Das Übertragungselement ist wenigstens abschnittsweise als flexibles Zugelement ausgebildet.

Wenigstens eine an dem Tragarm befestigbare Halteeinrichtung ist vorhanden, um z. B. einem Patienten eine Haltemöglichkeit zur Verfügung zu stellen. Dadurch ist es dem Patienten zum Beispiel möglich, sich an der Halteeinrichtung festzuhalten, hochzuziehen, sich umzulagern, zu positionieren oder ein Umlagern zu unterstützen. Der Tragarm kann insbesondere einfach verschwenkt werden, um unterschiedliche Arbeitspositionen zu ermöglichen.

Der Betätigungsmechanismus weist wenigstens eine Kraftrichtungseinheit auf, um eine (zwischen den Enden) in das flexible Zugelement eingeleitete Betätigungskraft gezielt zur Betätigung der Feststelleinrichtung zu nutzen bzw. zu der Feststelleinrichtung zu leiten. Die Kraftrichtungseinheit sperrt eine Kraftweiterleitung (durch das flexible Zugelement) in eine Richtung. Insbesondere wird eine Kraftweiterleitung radial nach innen gesperrt. Vorzugsweise ist eine Beweglichkeit des Zugelements radial nach innen hin sperrbar. Die Kraftrichtungseinheit umfasst wenigstens ein Anschlagteil, welches an dem Tragarm angeordnet ist. Bevorzugt ist wenigstens ein Anschlagteil in einem radialen Abstand zur Schwenkachse und/oder radial außerhalb der Befestigungseinrichtung angeordnet.

Da die Kraftrichtungseinheit eine Kraftweiterleitung in eine Richtung sperrt, kann die Kraftrichtungseinheit als Sperreinheit bezeichnet werden bzw. ist die Kraftrichtungseinheit als Sperreinheit ausgebildet.

Das flexible Zugelement umfasst insbesondere ein (wenigstens teilweise) seilartiges Element. Das Zugelement kann insbesondere wenigstens teilweise als Seil, Band, Kordel, flexibler Draht oder dergleichen ausgebildet sein oder wenigstens ein solches Element umfassen. Vorzugsweise ist das flexible Zugelement wenigstens bereichsweise elastisch ausgebildet. Es kann auch in gewissen Grenzen plastisch verformbar sein. Vorteilhaft umfasst das flexible Zugelement wenigstens teilweise Nylon oder auch ein anderes synthetisches Material und insbesondere Nylonfasern, und/oder auch Metall und insbesondere metallische oder auch synthetische Fasern oder Stränge und/oder auch Naturfasen wie Baumwolle, Leinen oder dergleichen. Das flexible Zugelement ist insbesondere als Zugseil, Band, Gummiband, Faden und/oder als Kette ausgeführt.

Das Übertragungselement kann auch mehrteilig ausgeführt sein.

Wenigstens ein Bestandteil kann als starrer Stab ausgeführt sein oder einen solchen umfassen.

Die erfindungsgemäße Radiologiehalteeinrichtung hat viele Vorteile. Die Kraftrichtungseinheit ermöglicht es, eine zur Betätigung der Feststelleinrichtung (in das Zugelement) eingeleitete Betätigungskraft direkt zur Feststelleinrichtung zu leiten und die (eingeleitete) Betätigungskraft (gezielt) zur Betätigung der Feststelleinrichtung zu nutzen. Die Gefahr, dass die Feststelleinrichtung, besonders beim Umlagern von Patienten, nicht oder nur mit einem sehr langen und/oder undefinierten Betätigungsweg betätigbar ist, wird minimiert oder sogar vollständig ausgeschlossen.

Besonders vorteilhaft ist es durch die Kraftrichtungseinheit möglich, die Betätigungskraft zur gezielten Betätigung der Feststelleinrichtung an einer beliebigen Position bzw. an Stellen entlang des Übertragungselements und insbesondere des Zugelements einzuleiten. Die Kraft wird durch die Kraftrichtungseinheit (bzw. Sperreinheit) in die gewünschte Richtung umgelenkt und/oder gerichtet. Ein als Seil ausgebildetes Zugelement kann nicht aus einer Führung herausgezogen werden. Ein ungewolltes Durchhängen des flexiblen Zugelements wird verhindert. Ein im Wesentlichen definierter Betätigungsweg resultiert daraus.

Das flexible Zugelement bzw. das Übertragungselement kann in größerer horizontaler Entfernung zur Feststelleinrichtung angeordnet werden, insbesondere in einer Entfernung, die größer ist als die Spannweite eines Arms des Benutzers, Auch bei einer langen horizontalen Führung des flexiblen Zugelements, d. h. insbesondere mit einem wesentlichen Abstand zur Schwenkachse, wird der Anteil der eingeleiteten Zugkraft bzw. der Betätigungsweg minimiert, welche nicht zur Betätigung des Feststellmechanismus beitragen.

Auch bei einem Durchhängen des flexiblen Zugelements, mehreren Zugelementen und/oder Betätigungselementen kann ein definierter Betätigungsweg (unabhängig von der Betätigungsposition entlang des Zugelements) eingestellt werden. Es muss jedenfalls nicht der durchhängende Bereich radial hinter (bzw. weiter vom Drehpunkt entfernt) der Betätigungsstelle (erst) stramm gezogen werden, bevor eine Betätigungskraft ausgeübt wird.

Die Kraftrichtungseinheit (bzw. Sperreinheit) bewirkt wenigstens ein lokale Sperrung der Bewegung in (wenigstens) eine Richtung. Dadurch wird eine Bewegung eines Abschnittes des flexiblen Zugelements nach innen hin verhindert, wenn der Betätigungspunkt radial weiter innen als die Kraftrichtungseinheit angeordnet ist. Das führt dazu, dass bei Betätigung die Feststelleinrichtung unmittelbar betätigt wird und nicht etwa erst ein längerer und z. B. durchhängender Abschnitt des flexiblen Zugelements gespannt und von radial weiter außen nach innen bewegt wird, bevor schließlich eine Betätigung der Feststelleinrichtung erfolgt. Das erhöht die Zuverlässigkeit und Sicherheit bei der Bedienung.

Eine Position des flexiblen Zugelements und des Betätigungselements zur Betätigung sind im Wesentlichen auch bei wiederholter Betätigung gleichbleibend. Dadurch werden die Arbeit von Pflegern bzw. dem Bedienpersonal und der Komfort der Patienten deutlich erhöht. Eine intuitive Bedienung ist möglich, da das Betätigungselement immer an der (im Wesentlichen) gleichen Position vermutet werden kann und/oder angeordnet ist.

Insgesamt wird die Bedienbarkeit der Radiologiehalteeinrichtung, und insbesondere des Tragarms, verbessert. Die Zuverlässigkeit des Betätigungsmechanismus wird dadurch deutlich verbessert.

Durch die definierte Positionierung des Übertragungselements und des flexiblen Zugelements, werden diese auch vor einer übermäßigen Verschmutzung geschützt. Das Betätigungselement wird vorteilhaft immer an der gleichen Stelle gegriffen. Dadurch wird insbesondere die Hygiene der Radiologiehalteeinrichtung unterstützt und gefördert. Darüber hinaus ist der Betätigungsmechanismus vor allem einfacher bedienbar. Ein Einsatz wird erleichtert.

Es ist wenigstens eine (als Sperreinheit ausgebildete) Kraftrichtungseinheit umfasst. Die Kraftrichtungseinheit ermöglicht es, eine zur Betätigung der Feststelleinrichtung eingeleitete Betätigungskraft direkt zur Feststelleinrichtung zu leiten und die Betätigungskraft und den Betätigungsweg (nahezu) vollständig zur Betätigung der Feststelleinrichtung zu nutzen. Eine Kraftrichtungseinheit leitet die auf das Betätigungselement ausgeübte Betätigungskraft in eine bevorzugte Richtung. Das bedeutet, dass das Übertragungselement in eine (entgegengesetzte) Vorzugsrichtung bewegt wird. Werden mehrere Betätigungselemente an unterschiedlichen Positionen hintereinander vorgesehen und wird ein Betätigungselement in der Mitte (zwischen den Enden) betätigt, so bewirkt die Kraftrichtungseinheit die Krafteinleitung im Wesentlichen nur in eine Richtung. Eine Bewegung des Übertragungselementes erfolgt (im Wesentlichen) in eine Richtung. Eine Bewegung in entgegengesetzter Richtung wird (weitgehend) verhindert. Dadurch wird der nötige Betätigungsweg verringert, da die Kraftrichtungseinheit die Kraft und den Weg zur Betätigung der Feststelleinrichtung gezielt ausrichtet und effektiv in die gewünschte Richtung lenkt.

Eine Drehachse des Tragarms ist im bestimmungsgemäß aufgebauten Zustand bzw. gebrauchsfertigen Zustand wenigstens im Wesentlichen vertikal oder im Wesentlichen etwa vertikal oder (nahezu oder exakt) vertikal oder horizontal ausgerichtet. Die Befestigungseinrichtung ist bevorzugt als Befestigungskonsole ausgebildet, welche insbesondere an der Decke eines Raumes z. B. einer Radiologieabteilung befestigt wird. Die Befestigungseinrichtung kann insbesondere auch als Wandbefestigung ausgeführt sein, welche sich an einer z. B. im Wesentlichen vertikalen Wand des Raums abstützt.

Die Halteeinrichtung ist insbesondere als Haltegriff ausgeführt oder umfasst einen solchen. Die Halteeinrichtung (bzw. der Haltegriff) ist insbesondere höhenverstellbar und kann so an kleinere und größere Personen angepasst werden. Vorzugsweise ist die Halteeinrichtung oder wenigstens ein Haltegriff um wenigstens 100 mm und insbesondere um 200 mm oder um 300 mm und z. B. auch um 500 mm oder um 1 m oder mehr in der Höhe verstellbar.

Das Tragteil bzw. dessen Unterseite ist vorzugsweise in einer Höhe von ca. 2000 mm vom Boden oder 2250 mm oder mehr angeordnet. Die insbesondere verstellbare Halteeinrichtung kann vorzugsweise in einer Höhe von 2000 mm oder mehr angeordnet sein. Eine größere Höhe stellt mehr Kopffreiheit zur Verfügung.

Die Befestigungseinrichtung kann auch direkt an dem Radiologiegerät angeordnet und/oder insbesondere darin integriert und/oder daran befestigt sein. Die Orientierung der Drehachse des Tragarms kann in diesem Fall und auch anderen Fällen durch einen Adapter anpassbar sein. Die Betätigungseinrichtung kann auch an einer Wand oder auf dem Fußboden befestigt oder in sonstiger Weise an dem Fußboden bzw. Boden angeordnet sein.

Der Tragarm ist bevorzugt drehbar, insbesondere wenigstens schwenkbar, an wenigstens der Befestigungseinrichtung der Radiologiehalteeinrichtung angeordnet und/oder aufgenommen und vorzugsweise befestigt. Insbesondere bewegt sich das Übertragungselement wenigstens abschnittsweise im Wesentlichen axial entlang der Drehachse oder dicht benachbart zu der Drehachse bei der Betätigung des Betätigungsmechanismus. Vorzugsweise bewegt sich das Übertragungselement wenigstens abschnittsweise zentral durch das Achsteil des Tragarms.

Das Übertragungselement ist vorzugsweise aus einem nicht magnetisch und/oder nicht elektrisch leitenden Material ausgebildet. Dadurch wird eine Antennenwirkung durch das Übertragungselement vermieden. Dies gilt insbesondere im Bereich der Durchführung durch das Achsteil und insbesondere durch die Schirmung und in den unmittelbar angrenzenden Bereichen bzw. der nahen Umgebung.

Ein Achtsteil und/oder ein Tragteil ist jeweils bevorzugt wenigstens teilweise und/oder abschnittsweise entlang der jeweiligen Längsachse als (umfänglich geschlossenes) Rohrteil mit einem wenigstens teilweise und/oder abschnittweise kreisförmig und/oder ovalen und/oder eckigen Querschnitt ausgeführt. Der Querschnitt kann insbesondere rund, 3-eckig, 4-eckig oder mehreckig, ausgebildet sein. Ein Achsteil und/oder ein Tragteil können auch wenigstens abschnittsweise und/oder wenigstens teilweise als offenes U-Profil und/oder V-Profil ausgebildet sein. Die teilweise Ausführung als Rohrteil und/oder offenes Profil bietet eine sehr robuste und verwindungssteife Konstruktion. Es ermöglicht eine gute Krafteinleitung und Kraftweiterleitung und einen einfachen und robusten Aufbau. Wenigstens das Achsteil ist wenigstens im Bereich der drehbaren Lagerung (oder über dem wesentlichen Teil der Länge oder der gesamten Länge) des Achsteils an der Befestigungseinrichtung rund ausgebildet.

Das Achsteil und/oder das Tragteil sind besonders bevorzugt aus einem nichtrostenden metallischen Werkstoff, insbesondere "VA-Stahl" oder einer anderen nichtrostenden Stahllegierung hergestellt. Insbesondere ist die Oberfläche des Werkstoffs so ausgebildet, dass auf einen Farbanstrich verzichtet werden kann. Dadurch wird verhindert, dass Farbe oder Farbpartikel bei mechanischer Beanspruchung abblättern und/oder abplatzen und/oder abgerieben werden und für Verschmutzung sorgen. Mögliche Verschmutzungen werden durch harte oder gehärtete Oberflächen und dadurch bedingte Vermeidung von Farbabrieb minimiert und/oder treten erst gar nicht auf.

Die Radiologiehalteeinrichtung ist insbesondere in Schwerkraftrichtung vorzugsweise mit einer Kraft von wenigstens 1350N oder 1500 N oder 1700 N oder 2000 N oder 3000 N belastbar. In einer Schwerlastausführung ist die Radiologiehalteeinrichtung vorzugsweise mit einer Kraft von bis zu 5000 N oder mehr belastbar.

Die Radiologiehalteeinrichtung nimmt insbesondere ein Aufnahmemoment von wenigstens 5,5 kNm oder wenigstens 6,5 kNm oder in einer Schwerlastausführung wenigstens 10,0 kNm oder ein noch höheres Aufnahmemoment auf. Die Radiologiehalteeinrichtung kann bevorzugt auch für die Aufnahme kleinerer oder größerer Kräfte und/oder Aufnahmemomente ausgelegt sein. Dies ist vor allem abhängig von der zu erwartenden Belastung. Falls vornehmlich oder nur Kinder oder besonders kleine und leichte Menschen verlagert werden sollen, kann die Belastungsgrenze deutlich geringer ausgelegt werden. Bei speziellen Radiologieanlagen für stark Übergewichtige kann die Radiologiehalteeinrichtung auch für entsprechend deutlich höhere Belastungen ausgelegt werden.

Besonders vorteilhaft ist der Betätigungsmechanismus der Radiologiehalteeinrichtung durch wenigstens zwei Betätigungselemente betätigbar. Insbesondere sind die Betätigungselemente wenigstens teilweise unabhängig voneinander betätigbar, d. h. dass die Betätigung der Feststelleinrichtung allein durch wenigstens ein Betätigungselement erfolgen kann. In einfachen Fällen kann wenigstens ein Teil oder Abschnitt eines Übertragungselementes durch ein Seil oder einen Faden oder dergleichen gebildet werden. Wenigstens ein Betätigungselement kann besonders bevorzugt als Knauf und/oder Knopf und/oder Griff ausgebildet sein oder ein solches Formelement umfassen. Möglich ist es auch, dass ein Betätigungselement durch eine Seilschlaufe und/oder ein Seilende gebildet wird. Auch andere geometrische Formen oder Körper sind möglich. Insbesondere können auch 3, 4, 5 oder mehr Betätigungselemente vorhanden sein. Vorteilhaft sind die Betätigungselemente an wenigstens einem Übertragungselement angeordnet und/oder wenigstens bereichsweise ausgebildet. Vorteilhaft ermöglicht das Betätigungselement eine komfortable und insbesondere sichere Bedienbarkeit des Betätigungsmechanismus. Dann kann eine Bedienperson z. B. an dem Betätigungselement ziehen und darüber an dem z. B. als Seil ausgeführten Übertragungselement ziehen und dadurch die Feststelleinrichtung lösen und somit gleichzeitig mit dem Betätigen auch den Tragarm in eine gewünschte Position verschwenken.

Außerdem ist insbesondere wenigstens ein Betätigungselement in der Nähe der Halteeinrichtung an dem Tragteil des Tragarms angeordnet. Vorteilhaft wird hierdurch eine Betätigung direkt an der Halteeinrichtung ermöglicht. Dadurch wird insbesondere der Komfort einer Betätigung erheblich verbessert.

Weitere Betätigungselemente können auch als elektrischer Sensor und/oder Schalter ausgeführt werden. In diesem Fall wird die Feststelleinrichtung über den Betätigungsmechanismus elektrisch betätigt. Ein Übertragungselement ist in diesem Fall bevorzugt durch ein elektrisch leitendes Kabel ausgebildet und/oder umfasst ein solches. Vorzugsweise wird aber kein motorisch oder elektrisch angetriebener Aktor verwendet, sondern mechanische Elemente.

Vorzugweise ist der Betätigungsmechanismus durch wenigstens zwei voneinander unabhängige Betätigungselemente betätigbar. Insbesondere sind mehr als zwei Betätigungselemente vorhanden, durch die der Betätigungsmechanismus separat betätigbar ist. Dabei können wenigstens jeweils zwei Betätigungselemente in Reihen- und/oder Parallelschaltung an dem Übertragungselement angeordnet bzw. angeschlossen sein. Die Betätigungselemente können so ausgerichtet werden, dass eine Betätigung vorteilhaft aus verschiedenen Positionen am Tragarm möglich ist. Ein Betätigungselement kann auch durch das Übertragungselement selbst gebildet werden. Dazu kann ein Betätigungselement durch einen in etwa horizontal verlaufenden Abschnitt des Übertragungselements gebildet werden.

In wenigstens einer vorteilhaften Weiterbildung umfasst der Betätigungsmechanismus wenigstens zwei (jeweils als Sperreinheit ausgebildete) Kraftrichtungseinheiten. Insbesondere können auch 3, 4, 5 oder noch mehr Kraftrichtungseinheiten umfasst sein. Vorteilhaft ist je Betätigungselement wenigstens eine Kraftrichtungseinheit umfasst. Außerdem kann es vorteilhaft sein, bei einem langen Zugelement bzw. Übertragungselement eine, zwei oder mehr Kraftrichtungseinheiten vorzusehen, um eine zuverlässige Betätigung der Feststelleinrichtung zu gewährleisten. Vorteilhaft wird durch mehrere Kraftrichtungseinheiten ein ungewolltes Durchhängen oder auch Dehnen des Übertragungselements und insbesondere des Zugelements verhindert bzw. kompensiert. Dadurch wird eine zuverlässige Betätigung mit einem im Wesentlichen definierten Betätigungsweg ermöglicht und noch weiter verbessert.

Vorzugsweise ist der Betätigungsweg des Zugelements kleiner als ein doppelter Durchmesser des Betätigungselements. Vorteilhaft kann der Betätigungsweg auch wenigstens kleiner als der vierfache, achtfache oder sogar zwanzigfache Durchmesser des Betätigungselements sein.

In wenigstens einer vorteilhaften Weiterbildung entspricht der Betätigungsweg an wenigstens einem Betätigungselement im Wesentlichen dem minimal notwendigen Weg zur Betätigung des Betätigungsmechanismus der Feststelleinrichtung. Das ist der minimale Betätigungsweg. Vorzugsweise ist ein Betätigungsweg an wenigstens einem anderen Betätigungselement nicht größer als der fünffache und insbesondere dreifache oder doppelte minimale Betätigungsweg.

Ein Hub bzw. der (minimale) Betätigungsweg des Übertragungselements beträgt vorzugsweise weniger als 100 mm und insbesondere zwischen 50 mm und 5 mm und vorzugsweise etwa 15 mm. Darüber hinaus kann der Betätigungsweg auch bis zu 30 mm, 60 mm und sogar bis zu 120 mm oder noch mehr betragen. Vorteilhaft kann durch einen kurzen und definierten Betätigungsweg eine Handhabung der Radiologiehalteeinrichtung verbessert werden. Ein Benutzer kann auch bei wiederholter Betätigung von einem definierten Betätigungsweg ausgehen. So können vorteilhaft auch Unfälle vermieden werden.

Vorzugsweise unterscheidet sich der zur Betätigung nötige Betätigungsweg von wenigstens zwei oder nahezu allen oder allen unterschiedlichen Betätigungselementen um weniger als 150 mm oder 100 mm und vorzugsweise um weniger als 50 mm und besonders bevorzugt um weniger als 25 mm.

Vorzugsweise unterscheidet sich der zur Betätigung nötige Betätigungsweg von wenigstens zwei oder nahezu allen oder allen unterschiedlichen Betätigungselementen um weniger als 300% oder 200% und vorzugsweise um weniger als 100% und besonders bevorzugt um weniger als 50% oder 25%. Bei kleineren (absoluten) Betätigungswegen kann die prozentuale Abweichung größer sein als bei größeren Betätigungswegen.

In einer konkreten Ausgestaltung liegt der Betätigungsweg an einem ersten Betätigungselement bei 15 mm und kann je nach Ausbildung und Einstellung um bis zu 10 mm größer oder kleiner sein. Die Abweichung zu einem Betätigungsweg eines zweiten Betätigungselement oder zu den Betätigungswegen (nahezu) aller anderen Betätigungselementen beträgt dann insbesondere weniger als 75 mm oder 50 mm und vorzugsweise weniger als 20 mm oder vorzugsweise weniger als 200% des Betätigungswegs. Beträgt der Betätigungsweg hingegen im Normalfall schon 100 mm so liegt eine Abweichung zu einem Betätigungsweg eines zweiten Betätigungselements insbesondere unter 100 mm und vorzugsweise unter 50 mm.

Die Kraftrichtungseinheit umfasst wenigstens ein Halteteil bzw. Anschlagteil. Der Begriff "Anschlagteil" wird hier synonym mit dem Begriff "Halteteil" verwendet und kann durchgängig in dieser Anmeldung dadurch ersetzt werden. Umgekehrt kann in dieser Anmeldung der Begriff Anschlagteil auch durch den Begriff Halteteil ersetzt werden. Im Folgenden wird der Begriff Anschlagteil verwendet. Das Anschlagteil ist an dem Tragarm angeordnet. Vorzugweise ist das Zugelement an dem Anschlagteil wenigstens bereichsweise aufgenommen und insbesondere wenigstens geführt und/oder sogar befestigt. Das oder wenigstens ein Anschlagteil ist insbesondere an dem Achsteil und/oder besonders bevorzugt an dem Tragteil des Tragarms angeordnet. Insbesondere umfasst, umschließt oder führt das Anschlagteil das flexible Zugelement. Das Anschlagteil kann das flexible Zugelement auch umlenken. In diesem Fall ist kein separates Führungselement zur Führung des flexiblen Zugelements notwendig. Vorzugsweise weist das Halteilteil zur Führung einen glatten Konturverlauf ohne Ecken und Kanten auf. Darüber hinaus kann das Anschlagteil sich auch an umgebenden Bauteilen oder Gegenständen abstützen oder daran angeordnet sein. Vorteilhaft ermöglicht das Anschlagteil, dass die eingeleitete Betätigungskraft im Wesentlichen direkt zu der Feststelleinrichtung geleitet wird.

In wenigstens einer vorteilhaften Ausgestaltung weist das Anschlagteil einen sich mit steigendem radialen Abstand von einer Schwenkachse aufweitenden Konturverlauf auf. Vorteilhaft wird durch den sich aufweitenden Konturverlauf eine Führung und eine Umlenkung des flexiblen Zugelements ermöglicht. Darüber hinaus wird eine gute Kraftumlenkung ermöglicht, sodass die Betätigungskraft direkt auf die Feststelleinrichtung wirken kann. Vorzugsweise ist wenigstens ein Anschlagteil in einem radialen Abstand zur Schwenkachse angeordnet, welcher wenigstens 20 % einer maximalen radialen Länge des Tragarms entspricht. Vorteilhaft ist wenigstens ein Anschlagteil auf einem radialen Abstand angeordnet welcher, der wenigstens 30 %, 50 %, 70 % oder einem noch größeren Anteil der maximalen Länge des Tragarms entspricht. Insbesondere ist das Anschlagteil am radialen Ende des Tragarms angeordnet. Wenigstens ein Anschlagteil ist radial außerhalb der Befestigungseinrichtung angeordnet. Vorteilhaft ist ein Betätigungselement in der Nähe der Halteeinrichtung angeordnet. Vorteilhaft ist so die Betätigung der Radiologiehalteeinrichtung besonders komfortabel möglich. Darüber hinaus ist eine Verschwenkung des Tragarms an einer radialen Position vorteilhaft möglich, welche einen besonders langen Hebelarm zur Schwenkachse aufweist, sodass ein Benutzer nur einen geringen Kraftaufwand hat.

Besonders bevorzugt ist wenigstens ein Betätigungselement, durch welches die Feststelleinrichtung betätigbar ist, radial außerhalb der Befestigungseinrichtung angeordnet und/oder befestigt. Vorteilhaft ist ein Betätigungselement insbesondere direkt benachbart zu wenigstens einer Halteeinrichtung angeordnet. Eine Halteeinrichtung kann bevorzugt in der Nähe des radialen Endes oder am radialen Ende, d. h. bei einem großen Radius des Tragarms angeordnet werden. Vorteilhaft kann eine Betätigung auch aus der Nähe einer Halteeinrichtung erfolgen.

Vorzugsweise weist die Kraftrichtungseinheit wenigstens ein Abstützteil auf. Das Abstützteil ist vorzugsweise an dem flexiblen Zugelement befestigt oder angeordnet. Besonders vorteilhaft umschließt oder umfasst das Abstützteil das flexible Zugelement wenigstens teilweise. Vorzugsweise dient das Abstützteil zum Abstützen und insbesondere zum Spannen des flexiblen Zugelements. Vorzugsweise kann dadurch eine im Wesentlichen definierte Lage des Zugelements und insbesondere des Betätigungselements erreicht werden. Das Abstützteil erfüllt vorteilhaft die Funktion eines "Seilstoppers", welche das insbesondere als Seil ausgebildete flexible Zugelement wenigstens bereichsweise vorspannt und dessen Bewegung in eine ungewollte Richtung sperrt. So kann die Betätigungskraft im Wesentlichen direkt zur Feststelleinrichtung geleitet werden. Deshalb kann das Abstützteil auch als Sperrteil bezeichnet werden, da es eine Bewegung des flexiblen Zugelements in eine Richtung sperrt. Der Begriff "Sperrteil" wird deshalb hier synonym mit dem Begriff "Abstützteil" verwendet und kann durchgängig in dieser Anmeldung dadurch ersetzt werden. Umgekehrt kann in dieser Anmeldung der Begriff Sperrteil auch durch den Begriff Abstützteil ersetzt werden. Im Folgenden wird der Begriff Sperrteil verwendet.

Das Sperrteil ist insbesondere (wenigstens bereichsweise kraftschlüssig und/oder formschlüssig) an dem Zugelement befestigt oder aufgenommen oder angeordnet. In wenigstens einer vorteilhaften Weiterbildung ist das Sperrteil über wenigstens einen Knotenabschnitt und/oder Schlaufenabschnitt des flexiblen Zugelements und/oder eine Schraubeneinheit mit dem Sperrteil verbunden und insbesondere daran aufgenommen.

Das Sperrteil kann auch durch eine (lokale) Verdickung des Zugelements gebildet werden. Das Sperrteil kann z. B. auch als Knoten ausgebildet sein oder als Querschnittsvergrößerung mit einem in oder an dem flexiblen Zugelement aufgenommenen fest oder elastisch ausgebildeten Verdickungskörper.

Besonders bevorzugt ist das Sperrteil an dem Zugelement befestigt und eine Bewegbarkeit des Sperrteils ist gegenüber dem Zugelement in wenigstens eine Richtung begrenzt.

Vorteilhaft weist das Sperrteil wenigstens eine wenigstens teilweise verschließbare Aufnahmeeinheit auf. Das Zugelement ist vorzugsweise an der verschließbaren Aufnahmeeinheit angeordnet.

Vorteilhaft kann so eine dauerhaft haltbare Verbindung zwischen dem Sperrteil und dem flexiblen Zugelement erreicht werden. Insbesondere ist das Sperrteil an dem Zugelement festsetzbar. Vorzugsweise kann hierzu die Aufnahmeeinheit und insbesondere die Schraubeneinheit wenigstens eine Madenschraube umfassen, durch welche das Zugelement wenigstens teilweise an dem Sperrteil festsetzbar ist. Die Lage des Sperrteils an dem flexiblen Zugelement ist vorzugsweise frei einstellbar. Vorzugsweise ist die Verbindung lösbar und alle Bauteile können ausgetauscht werden. Dadurch ist auch ein besonders hoher Anspruch an die Hygiene umsetzbar. Vorteilhaft ist das Sperrteil wenigstens teilweise an dem Anschlagteil abstützbar. Vorzugsweise ist das Sperrteil an dem Anschlagteil aufnehmbar. Das Sperrteil ist dabei insbesondere an die Kontur und den Konturverlauf des Anschlagteils angepasst. So kann das Sperrteil einen sich verjüngenden Konturverlauf aufweisen, welcher an dem Anschlagteil aufnehmbar ist. Insbesondere ist das Sperrteil rotationssymmetrisch ausgebildet, sodass sich das Sperrteil an dem Anschlagteil zentriert. Vorteilhaft stütz sich das Sperrteil wenigstens bei einer Einleitung der Betätigungskraft an dem Anschlagteil ab. Vorteilhaft wird die in das flexible Zugelement eingeleitete Betätigungskraft so direkt an die Feststelleinrichtung durch das Zugelement weitergeleitet. Die Nutzung des Sperrteils gewährleistet besonders bevorzugt eine sichere und reproduzierbare Funktion der Kraftrichtungseinheit und des Betätigungsmechanismus.

In allen möglichen vorteilhaften Ausgestaltungen kann der Betätigungsmechanismus wenigstens zwei Übertragungselemente umfassen. Vorzugsweise sind die Übertragungselemente durch wenigstens eine, insbesondere lösbare, Kupplungseinheit verbunden. Die Kupplungseinheit kann die Übertragungselemente verlängern oder auch einen Wechsel zwischen verschiedenen Übertragungselementen ermöglichen. So kann vorteilhaft eine kraftschlüssige Verbindung durch die Kupplungseinheit hergestellt werden. Es ist auch möglich, dass beispielsweise auch ein als Stabelement ausgebildetes Übertragungselement mit einem flexiblen Zugelement durch die Kupplungseinheit zu verbinden.

Vorzugsweise kann die Kupplungseinheit wenigstens ein Hülsenteil umfassen. Vorteilhaft umfasst die Kupplungseinheit außerdem wenigstens eine Schraubeneinheit. Vorteilhaft sind die Übertragungselemente insbesondere als flexible Zugelemente ausgebildet. Bevorzugt umschließen das Hülsenteil und die Schraubeneinheit die Übertragungselemente wenigstens teilweise. Die Schraubeneinheit kann insbesondere als Madenschraube ausgebildet sein, durch welche das Übertragungselement wenigstens teilweise durchgeführt ist. Insbesondere ist an einem flexiblen Zugelement wenigstens ein Knotenabschnitt ausgebildet. Vorteilhaft ist das Zugelement durch den Knotenabschnitt an der Kupplungseinheit und vorzugsweise dem Hülsenteil und/oder der Schraubeneinheit aufgenommen. Durch die Ausbildung der Kupplungseinheit wird eine schnell austauschbare Verbindung mehrerer Übertragungselemente ermöglicht. Vorzugsweise ist für die Öffnung bzw. Schließung der Kupplungseinheit kein Werkzeug notwendig.

In wenigstens einer vorteilhaften Weiterbildung ist das Übertragungselement durch wenigstens einen hohlen Abschnitt des Achsteils durchgeführt.

Durch die Durchführung des Übertragungselements durch den hohlen Abschnitt des Achsteils ist die Radiologiehalteeinrichtung insgesamt und insbesondere der Tragarm besonders kleinbauend und platzsparend. Das Übertragungselement muss nicht um eine Drehachse des Tragarms herum mitrotieren, wenn der Tragarm geschwenkt wird. Folglich ist eine Durchführung durch eine magnetische Schirmung besonders einfach möglich. Es ist kein Schlitz, kein Langloch oder keine linienförmige Öffnung notwendig, um das Übertragungselement schwenkbar durch eine eventuelle Schirmung oder Trennwand hindurchzuführen. Die Abdichtung bzw. die Abschirmung gestaltet sich deutlich einfacher.

Bei einer möglichen Durchführung der Radiologiehalteeinrichtung durch eine abgehängte Decke mit zum Beispiel einer magnetischen Schirmung (Faraday'scher Käfig) beschränkt sich die Durchtrittsfläche auf die Querschnittsfläche des Achsteils. Dadurch muss die Schirmung nur über einen minimalen Querschnitt entsprechend geöffnet und/oder durchbrochen werden. Das entstehende Loch der Schirmung muss jedenfalls weniger aufwendig oder gegebenenfalls gar nicht abgedichtet werden.

Vorzugsweise wird durch die Feststelleinrichtung der Radiologiehalteeinrichtung eine kraftschlüssige und/oder formschlüssige Verbindung zwischen Tragarm und Befestigungseinrichtung hergestellt. Die Feststelleinrichtung kann insbesondere kraftschlüssig als Reibbremse und/oder Riemenbremse oder dergleichen ausgebildet sein, bei der die Feststellposition kraftschlüssig durch das Zusammenwirken von Reibbelägen und/oder einem Riemen auf der Aufnahme realisiert wird. Bevorzugt sind ein Reibbelag an der Aufnahme und ein Reibbelag an dem Tragarm aufgenommen oder (z.B. einstückig) ausgebildet. Der Tragarm wird in diesem Fall verdrehsicher in einer Feststellposition geklemmt bzw. festgesetzt. Die Feststellposition kann auch kraftschlüssig durch z. B. eine magnetische arbeitende Feststelleinrichtung realisiert werden. In dieser Ausführungsform wird die Feststelleinrichtung des Tragarms z. B. entweder direkt durch Magnetkräfte in der gewünschten Feststellposition gehalten oder eine magnetische Kraft wird genutzt, um ein Fixierelement in eine Feststellposition zu überführen und/oder zu halten. In diesen Fällen sind beliebige Feststellpositionen in beliebigen Winkelstellungen möglich.

Die Feststelleinrichtung kann aber besonders bevorzugt auch formschlüssig ausgebildet sein. Hierbei ist die Winkelposition durch ineinandergreifende formschlüssige Elemente festgestellt. Es ist möglich, die formschlüssige Verbindung durch ineinandergreifende Elemente oder durch eine Verzahnung oder eine andere formschlüssige Kontur auszubilden.

Vorteilhaft umfasst die Feststelleinrichtung wenigstens ein Fixierelement zum Feststellen der Feststelleinrichtung. Vorzugsweise ist das Übertragungselement von einer Feststellposition in eine Drehposition überführbar, in der eine Winkelstellung des Tragarms gegenüber der Befestigungseinrichtung veränderbar ist. Insbesondere ist der Tragarm in der Drehposition frei drehbar oder in einem vordefinierten Winkelbereich schwenkbar. Es sind besonders bevorzugt Rasterpunkte vorgesehen.

Vorzugsweise wird bei einer Betätigung eines Betätigungselements ein Anschlagteil, welches in einem größeren radialen Abstand angeordnet ist als das betätigte Betätigungselement, eine Bewegung des Übertragungselements mittels des Sperrteils gesperrt, sodass die Betätigungskraft im Wesentlichen auf das Fixierelement der Feststelleinrichtung wirkt. Damit wird eine einfache Sperreinheit realisiert.

Das Übertragungselement ist mit dem Fixierelement mittelbar und/oder unmittelbar kraftschlüssig und/oder formschlüssig verbunden. Das Übertragungselement kann auch als elektrischer Leiter und/oder Aktor ausgebildet sein oder wenigstens einen solchen umfassen. In diesem und auch in anderen Fällen kann wenigstens ein zusätzlicher Aktor oder Aktuator umfasst sein. Es kann zum Beispiel ein Stellmotor vorhanden sein, durch welchen das Fixierelement aus der Feststellposition in die Drehposition (und umgekehrt) überführbar ist.

Es ist möglich, dass die Radiologiehalteeinrichtung durch wenigstens einen Aktuator und insbesondere durch einen Motor und vorzugsweise Elektromotor geschwenkt und/oder gedreht wird. Die Radiologiehalteeinrichtung ist in diesem Fall insbesondere per Fernbedienung, welche über eine Funk- oder Kabelverbindung zum Aktuator verfügt, verschwenkbar. In diesem Fall ist die Radiologiehalteeinrichtung in bevorzugten Varianten ausschließlich durch den Aktuator verschwenkbar, wenn diese in einer sicheren Feststellposition festgestellt ist. Eine solche Sicherheitsfunktion ist bevorzugt durch wenigstens einen Sicherheitsschalter überbrückbar. Bei dessen Betätigung ist die Radiologiehalteeinrichtung auch dann verschwenkbar, wenn sich die Radiologiehalteeinrichtung nicht in einer Feststellposition befindet.

In allen Ausgestaltungen umfasst die Feststelleinrichtung vorzugsweise wenigstens ein Fixierelement, welches in wenigstens ein Rastelement einer Mehrzahl von Rastelementen eingreift. Ein Fixierelement ist insbesondere an der Befestigungseinrichtung ausgebildet und/oder angeordnet, um mehrere Feststellpositionen zur Feststellung zu ermöglichen. Ein Rastelement ist insbesondere als Vertiefung und vorzugsweise als Loch bzw. als Bohrung ausgebildet. Wenigstens ein Fixierelement ist insbesondere als Eingriffselement ausgebildet, welches eine formschlüssige Verbindung mit der Befestigungseinrichtung ermöglicht bzw. herstellt. Wenigstens ein Eingriffselement ist vorteilhaft als Bolzen ausgebildet, welcher formschlüssig in einer Feststellposition in ein angepasstes Loch in der Befestigungseinrichtung eingreift.

Ein Rastelement und ein Fixierelement wirken vorzugsweise derart zusammen, um die Feststellposition zu ermöglichen. Wenn das Rastelement und ein Fixierelement in Eingriff miteinander stehen, wird eine Drehung blockiert und wenn sie außer Eingriff sind, kann der Tragarm verschwenkt werden. Besonders bevorzugt ist ein Rastelement als Vertiefung ausgebildet und das Fixierelement weist einen Bolzen oder dergleichen auf, der in die Vertiefung eingeführt werden kann. Es ist auch eine umgekehrte Wirkweise möglich, bei der das Fixierelement z. B. eine Vertiefung aufweist, in welche das z.B. bolzenförmige Rastelement in der Feststellposition eingreift. Es ist auch möglich, dass das Fixierelement und das Rastelement als aufeinander angepasste Formteile ausgebildet sind, die formschlüssig (und/oder auch kraftschlüssig) ineinander greifen. Das Fixierelement als Bestandteil der Feststelleinrichtung ist bevorzugt an dem Tragarm angeordnet. Eine Mehrzahl von entsprechenden Rastelementen (Vertiefungen) ist bevorzugt an der Befestigungseinrichtung ausgebildet und/oder daran angeordnet.

Die Zuordnung der Bolzen und der Vertiefungen zu dem Tragarm und der Befestigungseinrichtung kann aber auch genau entgegengesetzt sein, falls dies konstruktiv erforderlich oder wünschenswert erscheint.

Die Ausbildung der Feststelleinrichtung mittels wenigstens einem Bolzen als Fixierelement bzw. als Eingriffselement, welcher bzw. welches in ein Loch eines Lochkreises eingreift, bietet den Vorteil, dass die Feststelleinrichtung besonders einfach und kostengünstig fertigbar ist. Die Konstruktion ist sehr robust. Die formschlüssige Verbindung kann Querkräfte und Drehmomente gut aufnehmen und den Tragarm zuverlässig in einer Feststellposition feststellen. Der Mechanismus kann zentral und platzsparend um die Drehachse des Tragarms angeordnet werden.

Besonders bevorzugt sind eine Mehrzahl von Vertiefungen und insbesondere Löcher und/oder Bohrungen über einen Umfang auf einer Scheibe oder z. B. einem Ring verteilt, insbesondere als (regelmäßiger) Lochkreis, welcher an der Befestigungseinrichtung angeordnet ist. Durch Schwenken des Tragarms kann der Bolzen des Tragarms in den verschiedenen Winkelstellungen festgestellt werden, indem der Bolzen in eines der über den Umfang des Lochkreises verteilten Löcher eingreift. Darüber kann der Tragarm in der gewünschten Feststellposition fixiert werden.

Die Bohrungen sind besonders bevorzugt äquidistant in einer Teilung von bevorzugt 5°, 10°, 15° und/oder insbesondere 30°, und/oder besonders bevorzugt 45° und/oder insbesondere 90° zueinander angeordnet. Auch andere Teilungen sind möglich. Es ist weiterhin möglich, dass die Bohrungen nicht in einer gleichmäßigen Teilung über den Umfang verteilt sind. So ist beispielsweise auch eine Mehrzahl von Vertiefungen auf einem kleinen Abschnitt des Umfangs möglich, während nur eine kleine Anzahl an möglichen Feststellpositionen auf einem entsprechenden anderen Abschnitt des Umfangs angeordnet ist.

In einer vorteilhaften Ausgestaltungsvariante sind die Vertiefungen (etwa kreisförmig) auf einem Durchmesser angeordnet, welcher größer ist als ein Durchmesser des Achsteils. Besonders bevorzugt sind die Vertiefungen kreisförmig auf einem Durchmesser angeordnet, welcher größer ist als der 1,5 fache oder der doppelte (Außen-) Durchmesser des Achsteils. Vorteilhaft kann dadurch der Eingriff eines Bolzens in eine Vertiefung besonders einfach realisiert werden. Die Feststelleinrichtung mit den kreisförmig angeordneten Vertiefungen und einem Fixierelement, welches als Bolzen, Kegel, Kegelstumpf oder dergleichen ausgebildet ist, sind besonders einfach zugänglich. Dies ist vor allem bei Wartungsarbeiten und Reparaturen von Vorteil. Die Feststelleinrichtung kann dadurch bevorzugt direkt benachbart zu dem Achsteil angeordnet werden.

Vorzugsweise umfasst die Radiologiehalteeinrichtung wenigstens eine Vorbelastungseinrichtung, durch welche das Eingriffselement in die Feststellposition vorbelastbar ist. Durch die Vorbelastungseinrichtung befindet sich die Feststelleinrichtung besonders bevorzugt regelmäßig oder (nahezu) zu jedem Zeitpunkt in der Feststellposition, wenn diese sich nicht durch den Betätigungsmechanismus in der Drehposition befindet. Eine Vorbelastung ist insbesondere durch wenigstens eine Vorspannfeder möglich, welche den Bolzen als Fixierelement in passenden Winkelposition in das Loch der zugehörigen Feststellposition drückt. Die Vorbelastungseinrichtung kann bevorzugt als Druckfeder, Spiralfeder, Schraubenfeder, Blattfeder oder anderes federndes Element ausgebildet sein oder wenigstens ein solches umfassen. Es ist auch möglich, die federnden Eigenschaften eines anderen Bauteils zur Vorbelastung des Fixierelements zu nutzen. Die Vorbelastungseinrichtung kann insbesondere auch als magnetischer oder pneumatischer Mechanismus ausgeführt sein.

Bei einer Bewegung in Drehposition rastet das Fixierelement durch die Vorbelastungseinrichtung vorzugsweise automatisch ein, wenn das Fixierelement bei der Drehbewegung mit einer Vertiefung in Eingriff kommt. Dadurch wird der Tragarm vorteilhaft regelmäßig bzw. stets in eine fixierte Winkelstellung vorbelastet.

Durch die Vorbelastung des Fixierelements in die Feststellposition ist der Tragarm der Radiologiehalteeinrichtung verdrehsicher in seiner Winkelstellung, bzw. der Feststellposition, festgestellt. An der Halteeinrichtung des Radiologiegeräts kann sich eine Person sicher hochziehen und/oder abstützen, sodass die Radiologiehalteeinrichtung verdrehsicher zum Verlagern einer Person nutzbar ist. Auch bei Erschütterungen und/oder ruckartigen Bewegungen an einem Bestandteil der Radiologiehalteeinrichtung ist der Tragarm in seiner Winkelstellung zuverlässig festgestellt und kann auch durch Impulse nicht bewegt werden.

Vorzugsweise weist das Achsteil wenigstens in einem mittleren Bereich entlang der Längsachse eine insbesondere quer und vorzugsweise senkrecht zu dem Achsteil verlaufende Blendeneinrichtung oder ein Abdeckteil auf. Die Blendeneinrichtung dient insbesondere dazu, eine Durchführung durch wenigstens eine Zwischendecke und/oder eine magnetischen Schirmung abzudecken oder abzudichten. In und/oder an einer Zwischendecke ist insbesondere eine magnetische Schirmung vorgesehen, welche die durch den Betrieb eines Radiologiegeräts ausgesendete Strahlung ausreichend abschirmt. Insbesondere schließt die Blendeneinrichtung die magnetische Schirmung, sodass ein sicherer und zuverlässiger Betrieb eines Radiologiegeräts möglich ist. Dazu können insbesondere entsprechende Materialien zur Schirmung in und/oder an der Blendeneinrichtung verarbeitet und/oder integriert sein. Durch eine Verbindung der Blendeneinrichtung mit wenigstens einer Zwischendecke kann diese möglicherweise zur zusätzlichen Abstützung des Achsteils dienen. Dadurch wird die Steifigkeit des Systems verbessert. Es können auch mehrere Blendeneinrichtungen an einem Achsteil angeordnet sein.

Eine Ausgestaltung ohne Blendeneinrichtung ist auch möglich. Diese Ausgestaltungsvariante ist dann besonders bevorzugt, wenn die Radiologiehalteeinrichtung in einem Raum ohne Zwischendecke und/oder an einer Wand installiert werden soll. Hier kann eine zusätzliche Abdeckeinrichtung vorgesehen werden, welche insbesondere die Befestigungseinrichtung und den Feststellmechanismus vollständig abdeckt.

Am Betätigungselement kann insbesondere zusätzlich eine Schutzhülse angeordnet sein, welche das Übertragungselement und den Betätigungsmechanismus vor Verschmutzung vor allem durch die Hände des Benutzers schützen. Die Schutzhülse ist besonders bevorzugt mit zylinderförmiger und/oder ringförmiger Gestalt ausgeführt. Es sind jedoch auch andere Ausgestaltungsformen möglich. Die Hülse kann vorzugsweise einfach das Übertragungselement umfassen. Falls ein angrenzendes Betätigungselement als Knopf aufgeführt ist, kann sich die Hülse dort einfach Abstützen, sodass diese sich selbst über die Schwerkraft positioniert. Die Hülse ist besonders bevorzugt aus einem Werkstoff oder einer Werkstoffkombination hergestellt, welcher antibakteriell wirkt und einfach zu reinigen ist. Der Werkstoff weist vorteilhaft eine glatte Oberfläche auf, sodass sich Schmutz festsetzen kann. Vorzugsweise weist die Schutzhülse eine Länge größer 5 mm und insbesondere größer 10 mm auf. Vorzugsweise ist die Schutzhülse zwischen 5 mm und 100 mm, insbesondere zwischen 10 mm und 60 mm lang ausgebildet.

In einer besonders vorteilhaften Variante ist das Übertragungselement aus dem Inneren des Achsteils zu einem Bereich radial außerhalb des Achsteils geführt. Das Übertragungselement wird dabei (besonders platzsparend) durch das Achsteil geführt. Dadurch muss das Übertragungselement nicht neben dem Achsteil separat durch eine separat abzudichtende Öffnung in der Zwischendecke geführt werden. Die notwendige Öffnung beschränkt sich somit auf das Achsteil und dessen Querschnitt. Das Übertragungselement muss nicht um das Achsteil mitrotieren, wenn der Tragarm verschwenkt. Das Übertragungselement verschwenkt im Wesentlichen zentral mit dem Achsteil.

Besonders bevorzugt ist das Übertragungselement über wenigstens ein Viertel oder wenigstens die Hälfte der Länge des Tragteils, insbesondere wenigstens zwei Drittel der Länge des Tragteils und/oder vorteilhaft wenigstens drei Viertel der Länge des Tragteils an dem Tragteil aufgenommen und/oder geführt. Dadurch kann ein Betätigungselement besonders bevorzugt in der Nähe einer Halteeinrichtung positioniert werden. Der Betätigungsmechanismus kann gegebenenfalls auch für einen Patienten betätigbar sein. Jedenfalls kann eine Bedienperson radial weit außen greifen, um an dem Betätigungselement zu ziehen und die Feststelleinrichtung zu entriegeln, während die Bedienperson gleichzeitig das Tragteil des Tragarms führt und verschwenkt
Vorzugsweise wird das Übertragungselement durch wenigstens eine Führungseinheit innerhalb des Achsteils und/oder wenigstens einmal von innen nach außen aus dem Achsteil herausgeführt. Das Übertragungselement ist besonders bevorzugt innerhalb des Achsteils durch eine Führungseinheit geführt, welche als Führungsschiene ausgebildet ist. Die Führungsschiene ist insbesondere als Rohrteil mit kreisförmigen und/oder ovalem und/oder mehreckigem Querschnitt, welche insbesondere 3-eckig, 4-eckig, 5-eckig und/oder mehr Ecken umfasst, ausgeführt.

Die Führungseinheit kann auch als offenes Profil mit U-förmigen und/oder V-förmigen Querschnitt ausgeführt sein. Die Führungseinheit führt das Übertragungselement besonders bevorzugt über wenigstens einen Abschnitt entlang der axialen Richtung innerhalb des Achsteils. Durch die Führungseinheit wird das Übertragungselement wenigstens einmal radial von innen nach außen und/oder wenigstens einmal radial von außen nach innen geführt.

Die Führungseinheit kann einstückig und/oder mehrstückig sein. Sie kann aus mehreren Materialien bestehen. An einer Seite, an welcher das Übertragungselement geführt wird, kann eine besonders reibungshemmende Oberfläche ausgebildet und/oder ein reibungshemmender Belag oder eine Beschichtung vorgesehen sein, welche die Beweglichkeit eines Übertragungselements erleichtert und verbessert, wie zum Beispiel Grafit. Es kann auch eine selbstschmierende Beschichtung als Öl und/oder Fett vorgesehen sein.

Die Führungseinheit ist vorteilhaft aus einem Kunststoff ausgeführt, welcher eine besonders glatte und gleitfähige Oberfläche für das Übertragungselement bietet. An einer anderen Seite und/oder Position kann die Führungseinheit ein Material umfassen, welches besonders geeignet ist, um die Führungseinheit innerhalb des Achsteils aufzuhängen oder anzuordnen, wie zum Beispiel Stahl.

Eine Ausführung des Übertragungselements als mechanisches Zugelement bzw. als Seil, Kette oder Band bietet den Vorteil, dass an dessen Ende ein Knopf und/oder Knauf als Betätigungselement befestigbar ist und dass dadurch der Betätigungsmechanismus betätigbar ist. Der Betätigungsmechanismus ist einfach und direkt betätigbar. Bei Betätigung kann ein flexibles Zugelement kleine Lageänderungen der Hand einer Bedienperson leicht kompensieren, sodass das Betätigungselement sich mit der Hand des Patienten und/oder einer Bedienperson mitbewegt. Die Richtung und/oder die Orientierung und/oder der örtliche Verlauf des Zugelements können für einen Benutzer vorteilhaft variabel angeordnet werden, sodass auch das Seilende direkt als Betätigungselement nutzbar ist.

Eine Ausführung des Übertragungselements als mechanisches Zugelement bietet außerdem den Vorteil, dass wenigstens zwei Betätigungselemente einfach in Reihenschaltung und/oder Parallelschaltung an das Übertragungselement anschließbar sind. So ist es vorteilhaft möglich, dass der Betätigungsmechanismus durch wenigstens zwei Betätigungselemente betätigbar ist. Ein flexibles Zugelement des Übertragungselements ist ebenfalls bevorzugt entlang des Tragarms und insbesondere entlang des Tragteils verlegbar bzw. verlegt. Das Übertragungselement kann auch selbst als Betätigungselement nutzbar sein. Verschiedene Betätigungselemente können radial zueinander beabstandet sein, sodass eine Betätigung aus verschiedenen Positionen entlang des Tragarms und insbesondere des Tragteils des Tragarms möglich ist.

Vorteilhaft ist das Übertragungselement durch wenigstens eine außerhalb des Achsteils und/oder wenigstens eine innerhalb des Achsteils angeordnete Führungsrolle umgelenkt und/oder geführt. Eine Umlenkrolle ist bevorzugt an dem Achsteil des Tragarms angeordnet. Dadurch kann ein flexibles Übertragungselement besonders vorteilhaft zu wenigstens einer Feststelleinrichtung geführt werden. Eine Bewegung eines Übertragungselements kann einfach und direkt in eine gewünschte Richtung umgelenkt werden. Durch die Bewegung der Umlenkrolle bei Betätigung des Betätigungsmechanismus kann die Richtung der Bewegung des Übertragungselements besonders reibungsarm geführt werden. Besonders bevorzugt ist wenigstens eine Umlenkrolle nahe zu dem Achsteil angeordnet, an welchem das Übertragungselement radial aus dem Achsteil geführt wird. Eine Umlenkrolle kann auch am Tragarm angeordnet sein und das flexible Zugelement des Übertragungselements entlang des Tragarms führen.

Besonders bevorzugt wird das Übertragungselement durch wenigstens eine Umlenkhülse umgelenkt. Die Umlenkhülse ist insbesondere als Rohrteil ausgeführt und weist bevorzugt einen kreisförmigen und/oder ellipsenförmigen und/oder vieleckigen Querschnitt auf. Dadurch kann die Richtung der Wirkung des Übertragungselements besonders bevorzugt auch innerhalb kleiner Radien wirksam angepasst werden. Die Umlenkhülse kann auch als wenigstens teilweise offenes Profil mit einem U-förmigen und/oder V-förmigen Querschnitt ausgeführt sein. Die Führung kann insbesondere genutzt werden, um die Orientierung des Übertragungselements innerhalb des hohlen Achsteils und bei Eintritt und Austritt aus dem Achsteil zu ändern.

In einer besonders bevorzugten Ausgestaltungsvariante ist das Übertragungselement axial wenigstens nach unten aus dem Achsteil herausgeführt. Die axiale Herausführung des Übertragungselements aus dem Achsteil ermöglicht es vorteilhaft, die Richtung eines flexiblen Zugelements umzulenken. Dadurch kann das Übertragungselement zentral unterhalb der Drehachse des Tragarms aus dem Achsteil austreten, sodass der Betätigungsmechanismus im Wesentlichen zentral betätigbar ist.

Eine Umlenkhülse ermöglicht vorteilhaft eine Umlenkung des Übertragungselements auch bei kleinen Radien und dort wo eine Umlenkrolle nicht angeordnet werden kann.

Vorteilhaft umfasst die Radiologiehalteeinrichtung wenigstens eine Begrenzungseinrichtung zur Begrenzung eines Schwenkwinkels. Die Schwenkwinkelbegrenzungseinrichtung kann besonders bevorzugt als Anschlag ausgeführt werden, welcher z. B. an der Befestigungseinrichtung angeordnet und/oder befestigt wird. Die Begrenzungseinrichtung zur Begrenzung des Schwenkwinkels bzw. die Schwenkwinkelbegrenzungseinrichtung dient dazu, den Schwenkwinkel des Tragarms besonders bevorzugt in einem Gesamtdrehwinkel zu begrenzen, z. B. auf einen Schwenkwinkel von 270°, oder 180°, oder 90°. Der konkrete Winkel hängt vom Einzelfall und den konkreten Bedingungen ab. Der Schwenkwinkel kann deshalb auch in einem kleineren Gesamtwinkel begrenzt werden, welcher sich aus der Anordnung in dem Aufstellraum und/oder der Teilung und der Anzahl möglicher Feststellpositionen ergibt. Eine Ausführung der Radiologiehalteeinrichtung ohne Begrenzungseinrichtung ist auch möglich.

Besonders bevorzugt umfasst die Schwenkwinkelbegrenzungseinrichtung ein Dämpferelement, welches einen weichen und gedämpften Anschlag ermöglicht. Das Dämpferelement ist vorzugsweise als Gummipuffer und/oder als Gasfeder und/oder als Gummizug ausgebildet. Dadurch kann eine Radiologiehalteeinrichtung insbesondere so angeordnet bzw. in einen Radiologieraum integriert werden, dass die Nutzung an die Anforderungen und die Bedürfnisse einer Radiologieanlage, eines Patienten oder der Bedienpersonen und die geometrischen Abmessungen des Raums angepasst werden kann. Vorzugsweise kann hierfür wenigstens ein Anschlag an der Feststelleinrichtung vorgesehen werden. Die Begrenzungseinrichtung kann insbesondere auch an der Wand des Raums angebracht sein. In diesem Fall ist die Begrenzungseinrichtung nicht in die Feststelleinrichtung integriert. Hierdurch wir ein Arbeitsbereich der Radiologiehalteeinrichtung definiert.

In einer besonders vorteilhaften Variante umfasst die Radiologiehalteeinrichtung eine Stoßschutzeinheit, welche die Radiologiehalteeinrichtung insbesondere großflächig und/oder insbesondere vollständig umschließt und gegen Stöße und Kollisionen mit anderen Geräten im Radiologieraum beim Verschwenken (weitgehend oder weitestgehend) schützt.

Solch eine Stoßschutzeinheit ist insbesondere geeignet, um die Radiologiehalteeinrichtung und/oder andere Komponenten der Radiologieanlage vor Beschädigungen im Betrieb zu schützen. Die Dämpfereinheit weist besonders bevorzugt einen (Außen-) Durchmesser von wenigstens 100 oder 150 mm, insbesondere von 250 mm und/oder 350 mm auf. Es sind jedoch in Abhängigkeit des zur Verfügung stehenden Bauraums und der Gegebenheiten des Radiologieraums auch kleinere Durchmesser möglich. Die Stoßschutzeinheit ist insbesondere am Tragteil des Tragarms ausgebildet und/oder umschließt diesen vollständig. Die Stoßschutzeinheit kann insbesondere auch den Achsteil des Tragarms wenigstens teilweise mit umschließen. Vorteilhaft liegt ein effektiver Schutz gegen Beschädigung vor.

Vorteilhaft umfasst die Stoßschutzeinheit einen elastischen Werkstoff, vorzugsweise Gummi und/oder Plastik. Das Dämpferelement kann besonders bevorzugt auch mehrere Materialien umfassen. Es ist auch möglich, dass die Stoßschutzeinheit besonders bevorzugt Verstärkungselemente aus einem Hartplastik oder metallischen Werkstoff umfasst.

Besonders vorteilhaft umfasst die Radiologiehalteeinrichtung wenigstens eine Aufnahmeschiene mit wenigstens einem Einhängepunkt oder einer Einhängestelle, in den wenigstens eine Halteeinrichtung einhängbar ist. Vorteilhaft erstreckt sich die Aufnahmeschiene über wenigstens einen wesentlichen Teil der radialen Länge des Tragarms. Die wenigstens eine Aufnahmeschiene ist entlang des Tragarms ausgebildet. Eine Aufnahmeschiene kann dabei so biegesteif ausgebildet sein, dass sich wenigstens über 25 %, 50 %, 75 % oder so bis zu 100 % der radialen Länge des Tragarms erstreckt. Die Länge einer Aufnahmeschiene kann dabei jedoch auch deutlich kürzer sein als die Länge des Tragarms. Insbesondere können auch mehrere Aufnahmeschienen entlang des Tragteils angeordnet sein. Dadurch ist die Anordnung besonders verwindungssteif und bietet ein hohes Widerstandsmoment bei Belastung mit Normal- und/oder Querkräften und/oder Biegemomenten.

Die Halteeinrichtung umfasst insbesondere wenigstens ein Einhängeelement zum Einhängen in die Aufnahmeschiene. Diese ist insbesondere als Hakenelement und insbesondere als Karabinerhaken oder auch als offener Haken ausgeführt oder umfasst insbesondere wenigstens ein solches. Bevorzugt sind bei einem offenen Hakenelement die Hakenschenkel so bemessen, dass sehr große Haltewinkel von 45° und mehr möglich sind.

Vorzugsweise ermöglicht die Halteeinrichtung einen Haltewinkel von wenigstens 45°. Vorteilhaft wird der Haltewinkel zur Vertikalen gemessen. Bevorzugt ist der Haltewinkel in Richtung eines Erdbeschleunigungsvektors geöffnet. Vorzugsweise sind Haltewinkel von bis zu 60° und mehr möglich. Besonders vorteilhaft wird so ein sicherer Halt in nahezu allen möglichen Lagen erreicht. Vorzugsweise kann ein als offener Haken ausgeführtes Hakenelement nur unter einem Schwenkwinkel von mehr als 30° und insbesondere von mehr 45° und besonders bevorzugt von mehr als 60° zur Vertikalen aus der Aufnahmeschiene entnommen werden. Dann kann der kurze Schenkel hochgeschoben und die Halteeinrichtung mit dem offenen Haken entnommen werden. Unter einem entsprechenden Winkel kann der offene Haken der Halteeinrichtung an einer anderen Stelle der Aufnahmeschiene oder einer anderen Aufnahmeschiene wieder eingeführt werden, um eine andere Position der Halteeinrichtung zu ermöglichen.

Insbesondere sind mehrere Einhängepunkte oder Einhängestellen entlang der Aufnahmeschiene angeordnet und/oder ausgebildet. Die Aufnahmeschiene ist besonders bevorzugt an beiden Enden mit dem Tragarm verbunden.

Insbesondere sind wenigstens zwei Aufnahmeschienen über die gesamte Länge des Tragarms angeordnet, sodass jeder Punkt in dem vornehmlich kreisförmigen Arbeitsbereich durch die Halteeinrichtung erreichbar ist.

Zur vorteilhaften Lagerung der Halteeinrichtung kann zusätzlich wenigstens ein Lagerungshaken z. B. am äußeren Ende des Tragarms oder an verschiedenen Längsstellen zur Lagerung der Halteeinrichtung (bei Nichtgebrauch) angeordnet sein. Durch das Einhängen der Halteeinrichtung an den Lagerungshaken ist die Halteinrichtung bei Schwenkbewegungen des Tragarms nicht hinderlich. Besonders vorteilhaft wird hierdurch eine einfache, sichere und leicht veränderbare Sicherung der Halteeinrichtung ermöglicht.

Ein Einhängepunkt oder eine Einhängestelle kann eine rastende Stellung der Halteeinrichtung zur Verfügung stellen. Die Funktion der Aufnahmeschiene ist nicht auf die Einhängevorrichtung begrenzt. Sie kann insbesondere auch zum Halten von Infusionen bzw. Infusionsbeuteln und/oder Infusionshalterungen genutzt werden. Auch eine Nutzung für andere Funktionen ist denkbar. Auch separate Infusionshalterungen sind möglich.

Die Aufnahmeschiene ist besonders bevorzugt aus einem nichtrostenden metallischen Werkstoff, insbesondere "VA-Stahl" oder einer anderen nichtrostenden Stahllegierung hergestellt. Insbesondere ist die Oberfläche des Werkstoffs so ausgebildet, dass ein Farbanstrich nicht nötig ist und dass bei mechanischer Beanspruchung nur wenig oder gar kein Abrieb entsteht. Die Aufnahmeschiene kann auch quer oder schräg zum Tragarm und insbesondere zum Tragteil ausgebildet sein. Es ist auch möglich zwei oder mehr Aufnahmeschienen an wenigstens einem Tragarm anzuordnen. Die Aufnahmeschiene ist vorteilhaft so ausgeführt, dass eine Halteeinrichtung zwischen mehreren verschiedenen Einhängepunkten bzw. Einhängestellen bewegt werden kann, ohne dass die Haltevorrichtung aus der Aufnahmeschiene insgesamt ausgehangen werden muss. Besonders bevorzugt sind zwei Aufnahmeschienen über die komplette radiale Länge des Tragarms ausgebildet. Ein Aushängen ist dann nur in dem Fall notwendig, wenn die Halteeinrichtung beispielsweise von einer Aufnahmeschiene in eine andere Aufnahmeschiene umgehängt werden soll. Denkbar sind auch drei oder mehr Aufnahmeschienen.

Die Einhängepunkte werden bevorzugt durch Formelemente gebildet, in welche ein Ringelement der Halteeinrichtung vorteilhaft bei Zugbelastung eingreift. Die Einhängepunkte bzw. Einhängestellen können durch lokale "Täler" an der Aufnahmeschiene gebildet werden, die durch lokale "Berge" dazwischen begrenzt werden. So kann die Position der Halteeinrichtung entlang des Tragarms verändert werden, ohne dass die formschlüssige Verbindung zwischen der Aufnahmeschiene und der Halteeinrichtung gelöst werden muss. Hierdurch kann die Position der Halteeinrichtung einfach und schnell zwischen einer Mehrzahl an Positionen angepasst und verändert werden. Dazu wird die Halteeinrichtung einfach in den nächsten Einhängepunkt weitergeschoben bzw. über den nächsten "Berg" gehoben. Die Halteeinrichtung muss dafür nicht aufwendig abmontiert werden, sodass diese bei Veränderung der Einhängeposition nicht herunterfallen kann. Eine Verletzungsgefahr für einen Benutzer ist minimiert.

Besonders vorteilhaft umfasst die Halteeinrichtung eine Gurteinheit. Die Gurteinheit ist besonders vorteilhaft zwischen dem Einhängeelement und dem Griffelement angeordnet. Die Gurteinheit dient vornehmlich dazu, einem Patienten das Aufrichten und Verlagern zu erleichtern. Dazu kann die Länge eines umfassten Gurtelements, insbesondere motorisch, an die Bedürfnisse und Gegebenheiten eines Benutzers angepasst werden.

Besonders bevorzugt ist die Gurteinheit im gebrauchsfertigen Zustand um eine quer zur Horizontalen ausgerichtete Achse drehbar gelagert. Besonders bevorzugt ist die Gurteinheit leichtgängig gelagert, sodass sich bei einer Verdrehung der Halteeinrichtung bzw. eines Haltegriffes der Halteeinrichtung die Gurteinheit um ihre Lagerstelle dreht und der Gurt selber nicht oder nur geringfügig tordiert. Vorzugsweise dreht sich die Gurteinheit erheblich mehr um ihre Lagerstelle als sich der Gurt an sich tordiert, wenn die Halteeinrichtung verschwenkt wird.

Der Gurt wird vorzugsweise durch ein breites Gewebeband gebildet. Vorteilhaft verdreht sich der Gurt im bestimmungsgemäßen Gebrauch nicht oder nur unwesentlich. Die Anpassung der Länge dient vor allem dazu, die Länge der Halteeinrichtung vorteilhaft an den Benutzer und dessen Lage anzupassen, sodass eine vorteilhafte Abstützung und/oder Verlagerung in verschiedenen Positionen möglich ist.

Ein motorischer Antrieb der Gurteinheit kann besonders bevorzugt durch eine Wickelfeder oder gegebenfalls auch über einen Elektromotor, insbesondere als Stellmotor ausgeführt, erfolgen. Die Steuerung kann dabei besonders vorteilhaft durch eine Steuerungseinheit erfolgen, welche direkt an der Halteeinrichtung oder durch eine Fernbedienung betätigbar und/oder steuerbar ist. Eine alternative Ausführung des motorischen Antriebs als Pneumatik- und/oder Hydraulikmotor ist auch denkbar.

In allen Ausgestaltungen ist es möglich, dass Patienten eine Umlagerung oder Verlagerung selbstständig unterstützen. Mit der Gurteinheit ist es außerdem besonders vorteilhaft möglich, dass sich Patienten mit Hilfe der Lagerungseinheit bzw. des Haltegriffs selbst angeben und die Umlagerung untersützen, So können Patienten von als Liege und/oder Stuhl und/oder Tragband ausgeführten Verlagerungseinheiten umgelagert werden. Es sind auch andere Arten und Ausführungsformen von Verlagerungseinheiten möglich.

Vorteilhaft wird die notwendige Unterstützung durch das Pflege- oder auch Radiologiepersonal beim Umlagern minimiert. Im Idealfall muss ein Pfleger im Wesentlichen gar nicht beim Umlagern unterstützen. Dadurch wird vor allem auch ein Übertragungsrisiko von Bakterien, Viren und Keimen minimiert. Das Potential für sexuelle Belästigungen bei oder während des Umlagerns wird deutlich minimiert. Arbeitsausfälle durch psychischen Stress oder auch durch übertragene Keime werden deutlich minimiert. Dadurch werden vor allem die Bedienbarkeit und auch die Wirtschaftlichkeit von Radiologieanlagen verbessert.

Vorteilhaft ist die Radiologiehalteeinrichtung wenigstens teilweise aus schwer und/oder nicht oder nur gering magnetisierbaren Werkstoffen gefertigt. Beim Betrieb von Radiologiegeräten können Magnetfelder mit hoher Intensität auftreten. Metallische Werkstoffe mit entsprechendem molekularem Aufbau könnten so magnetisiert werden, was zu einer Wechselwirkung mit dem Radiologiegerät führen könnte. Bevorzugt sind daher alle Komponenten und Bauteile der Radiologiehalteeinrichtung aus ausschließlich gering und insbesondere nicht magnetisierbaren Werkstoffen gefertigt, wie zum Beispiel Kunststoff und/oder Aluminium und/oder Edelstahl. Vorteilhaft sind wenigstens jedoch große Teile der Radiologiehalteeinrichtung wenigstens teilweise aus nicht oder nur schwer magnetisierbaren Werkstoffen gefertigt. Dadurch können magnetische Wechselwirkungen (und damit Artefakte bei der Aufnahme) zwischen der Radiologiehalteeinrichtung und dem Radiologiegerät ausgeschlossen und/oder wenigstens minimiert werden. Dies dient vor allem dem sicheren und zuverlässigen Betrieb des Radiologiegeräts und einer hohen Qualität der damit erstellten Aufnahmen.

In wenigstens einer vorteilhaften Weiterbildung ist wenigstens ein Übertragungselement wenigstens abschnittsweise durch einen hohlen Abschnitt des Tragteils geführt. Vorteilhaft kann auch eine Kraftrichtungseinheit wenigstens teilweise innerhalb des Tragteils angeordnet sein. So kann das Zugelement insbesondere hygienisch vor Verschmutzung geschützt werden.

Besonders bevorzugt umfasst eine erfindungsgemäße Radiologieanlage wenigstens ein Radiologiegerät und wenigstens eine Radiologiehalteeinrichtung.

Vorteilhaft umfasst die Radiologieanlage wenigstens eine Aufnahme für Patienten zur Untersuchung mit dem Radiologiegerät, welche wenigstens teilweise im Arbeitsbereich der Radiologiehalteeinrichtung angeordnet ist. Bevorzugt ist die Aufnahme für Patienten zentral im Arbeitsbereich der Radiologiehalteeinrichtung angeordnet, sodass der Arbeitsbereich der Radiologiehalteeinrichtung effektiv nutzbar ist.

Weitere Vorteile und Merkmale der vorliegenden Erfindung ergeben sich aus dem Ausführungsbeispiel, welches im Folgenden mit Bezug auf die beiliegenden Figuren erläutert wird.

Die Anmelderin behält sich vor, separaten Schutz für eine Geburtshilfehalteeinrichtung für eine Geburtshilfevorrichtung, wie insbesondere einen Gebärstuhl oder einen Gebärhocker, eine Gebärwanne oder ein Gebärbett zu beantragen, welche eine Befestigungseinrichtung und wenigstens einen daran schwenkbar aufgenommenen Tragarm umfasst, wobei der Tragarm wenigstens ein Achsteil und wenigstens ein Tragteil aufweist, wobei das Achsteil des Tragarms schwenkbar an der Befestigungseinrichtung aufgenommen ist, um den Tragarm zu verschwenken. Dabei umfasst die Geburtshilfehalteeinrichtung eine Feststelleinrichtung, um den Tragarm in wenigstens einer Feststellposition festzustellen und einen Betätigungsmechanismus mit wenigstens einem Übertragungselement und wenigstens einem Betätigungselement, um die Feststelleinrichtung zu betätigen, wobei das Übertragungselement wenigstens abschnittsweise als flexibles Zugelement ausgebildet ist. Die Geburtshilfehalteeinrichtung weist wenigstens eine an dem Tragarm befestigbare Halteeinrichtung auf, um z. B. einer Gebärenden eine Haltemöglichkeit z. B. beim Aufrichten oder Umlagern oder Halten zur Verfügung zu stellen. Dabei ist das Achsteil des Tragarms schwenkbar an der Befestigungseinrichtung aufgenommen, um den Tragarm zu verschwenken. Der Betätigungsmechanismus weist eine Kraftrichtungseinheit auf, um eine in das flexible Zugelement eingeleitete Betätigungskraft gezielt zur Betätigung der Feststelleinrichtung zu leiten. Insbesondere umfasst der Betätigungsmechanismus wenigstens ein durch einen hohlen Abschnitt des Achsteils durchgeführtes Übertragungselement. In Weiterbildungen kann die Geburtshilfehalteeinrichtung Merkmale umfassen, wie sie hier mit Bezug auf die Radiologiehalteeinrichtung beschrieben werden.

In den Figuren zeigen:
- Figur 1: eine Vorderansicht der Radiologieanlage mit einer Schnittansicht eines Ausführungsbeispiels einer erfindungsgemäßen Radiologiehalteeinrichtung und der Vorderansicht eines Radiologiegeräts;
- Figur 2: eine Draufsicht auf eine Radiologieanlage mit einem Ausführungsbeispiel einer erfindungsgemäßen Radiologiehalteeinrichtung und ein im Arbeitsbereich angeordnetes Radiologiegerät;
- Figur 3: eine vergrößerte Teilansicht des Achsteils des Ausführungsbeispiels einer erfindungsgemäßen Radiologiehalteeinrichtung;
- Figur 4: eine vergrößerte Schnittansicht einer Kraftrichtungseinheit eines Ausführungsbeispiels einer erfindungsgemäßen Radiologiehalteeinrichtung;
- Figur 5: eine Detailansicht einer Kupplungseinheit eines Ausführungsbeispiels einer erfindungsgemäßen Radiologiehalteeinrichtung;
- Figur 6: eine Detailansicht eines Sperrteils eines Ausführungsbeispiels einer erfindungsgemäßen Radiologiehalteeinrichtung;
- Figur 7: eine vergrößerte Schnittansicht durch ein Tragteil eines Ausführungsbeispiels einer erfindungsgemäßen Radiologiehalteeinrichtung;
- Figur 8: eine perspektivische Ansicht eines Ausführungsbeispiels einer Halteeinrichtung einer erfindungsgemäßen Radiologiehalteeinrichtung.

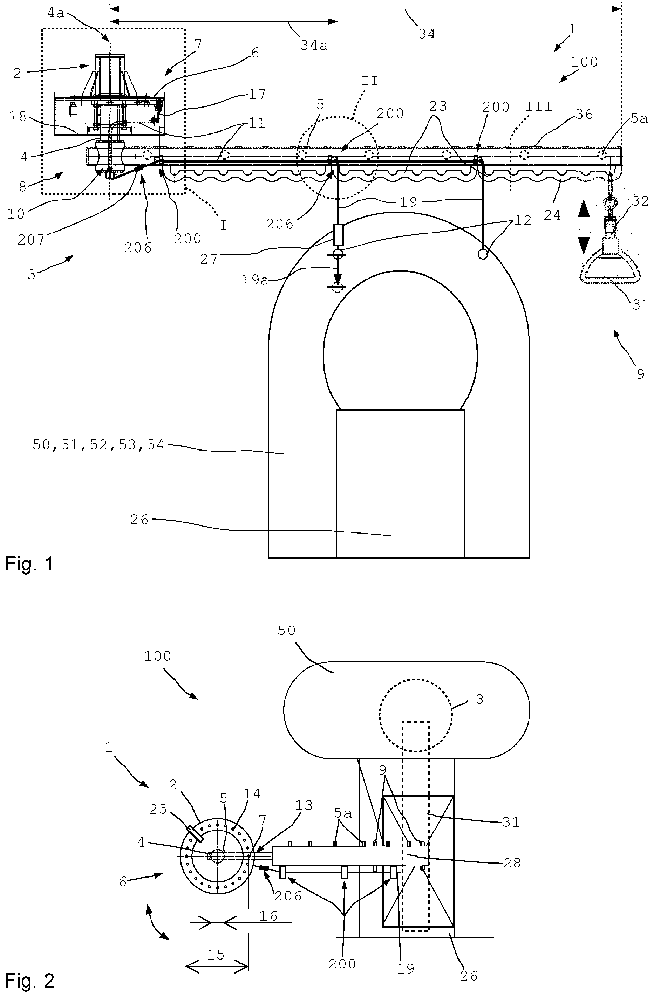

Figur 1 zeigt eine Ansicht der erfindungsgemäßen Radiologieanlage 100. Die Radiologiehalteeinrichtung 1 ist in einer Schnittdarstellung gezeigt, während das Radiologiegerät 50, wie ein Röntgengerät 51, ein MRT-Gerät 52, ein CT-Gerät oder ein Strahlentherapiegerät 54 in einer Vorderansicht dargestellt ist. Die Radiologiehalteeinrichtung 1 umfasst eine Befestigungseinrichtung 2, an welcher der Tragarm 3 schwenkbar aufgenommen und gelagert ist. Der Tragarm 3 umfasst ein Achsteil 4, welches an der Befestigungseinrichtung 2 schwenkbar befestigt ist, und ein Tragteil 5. Der Tragarm 3 ist um die Drehachse 4a bzw. Schwenkachse 4a verschwenkbar.

Die Befestigungseinrichtung 2 ist hier als Befestigungskonsole ausgeführt und an einer Decke eines nicht dargestellten Radiologieraums fest montiert. Die Blendeneinrichtung 18 ist mittig an dem Achsteil 4 angeordnet und deckt die Öffnung ab, welche durch die Durchführung des Tragarms 3 durch die magnetisch schirmende Zwischendecke vorhanden ist. Falls die Radiologiehalteeinrichtung 1 nicht durch eine Zwischendecke geführt ist, ist die Blendeneinrichtung 18 bevorzugt so ausgeführt, dass diese die Befestigungseinrichtung 2 vollständig umschließt.

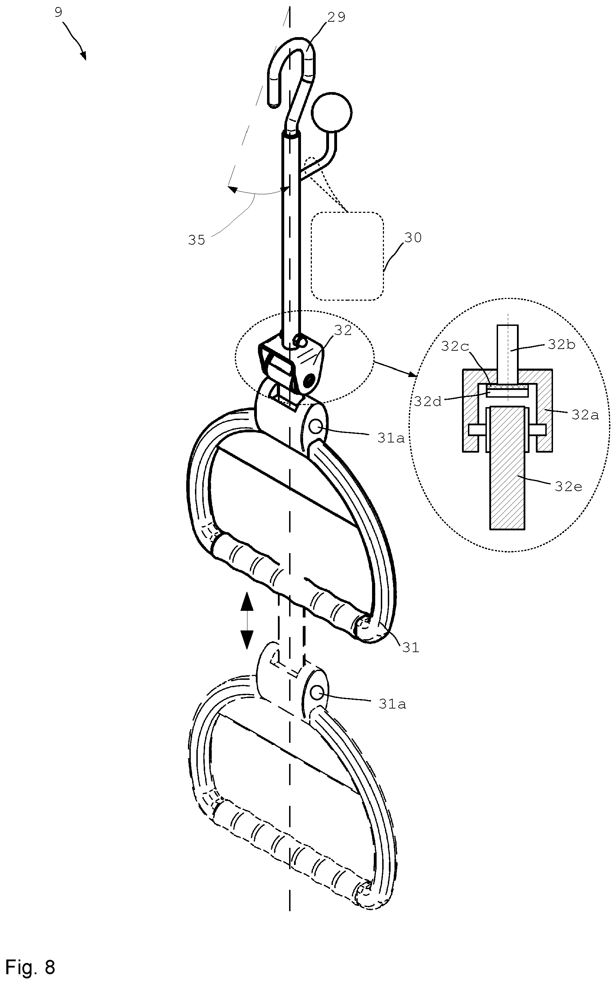

In die Einhängepunkte 24 ist eine Halteeinrichtung 9, welche durch einen Griff mit wenigstens einem Hakenelement 35 gebildet wird. Die Halteeinrichtung 9 ist detailliert in Figur 8 dargestellt und beschrieben.

Die Radiologiehalteeinrichtung 1 ist in der Nähe eines Radiologiegeräts 50, wie etwa einem Röntgengerät 51, einem MRT-Gerät 52, einem CT-Gerät 53 oder einem Strahlentherapiegerät 54 angeordnet. Der Tragarm 3 kann über den Arbeitstisch 26 für Patienten geschwenkt werden, sodass die Halteeinrichtung 9 für einen Patienten gut erreichbar ist.

Entlang des Tragteils 5 sind hier zwei Aufnahmeschienen 23 mit mehreren Einhängepunkten 24 über die gesamte Länge (34) des Tragteils 5 ausgebildet. In einem Einhängepunkt 24 der Aufnahmeschiene 23 ist eine Halteeinrichtung 9 eingehängt, an welcher sich ein Patient zum Verlagern und Positionieren abstützen kann. Ein Infusionsbeutel 30 kann auch in einen Einhängepunkt 24 der Aufnahmeschiene 23 eingehängt werden. Ein Einhängepunkt 24 der Aufnahmeschiene 23 ist hierzu als "Tal" zwischen zwei "Bergen" in der Kontur der Aufnahmeschiene 23 ausgebildet. Bei einer Belastung der Halteeinrichtung 9 auf Zug, wie beim bestimmungsgemäßen Gebrauch, ist die Position der Halteeinrichtung 9 in dem Einhängepunkt 24 formschlüssig durch die Geometrie des "Tals" fixiert. Die Halteeinrichtung 9 umfasst eine vorzugsweise mechanisch über eine Feder betriebene Gurteinheit 32, durch welche die Länge der Halteeinrichtung 9 variabel einstellbar ist. Die Halteeinrichtung 9 ist über wenigstens ein Hakenelement 29 an dem Einhängepunkt 24 aufgenommen. Zusätzlich ist es hier auch möglich, dass die Halteeinrichtung 9 über einen hier nicht dargestellten Lagerungshaken gelegt werden kann, sodass dieser beim Verschwenken des Tragarms 3 nicht stört.

Unmittelbar radial benachbart neben dem Griffknopf als Betätigungselement 12 sind an dem Tragteil 5 zwei Aufnahmeschienen 23 ausgebildet. Jede Aufnahmeschiene 23 umfasst mehrere Einhängepunkte 24. Die Einhängepunkte 24 sind als "Täler" in der Kontur der Aufnahmeschiene 23 ausgebildet. In die Einhängepunkte 24 ist eine Halteeinrichtung 9, welche durch einen Griff und zwei Hakenelementen gebildet wird, in den äußersten Einhängepunkt 24 eingehängt. Die einzelnen "Täler" der Aufnahmeschiene 23, welche als Einhängepunkte 24 dienen, sind durch "Berge" in der Kontur voneinander getrennt. Bei Zugbelastung, wie im bestimmungsgemäßen Gebrauch, wird die Position der Ringschlaufe der Halteeinrichtung 9 in dem Einhängepunkt 24 fixiert. Die Halteeinrichtung 9 kann einfach in einen anderen Einhängepunkt 24 über einen "Berg" geschoben werden, ohne dass ein Hakenschenkel ausgehangen werden muss. Hierdurch ist eine sichere und belastbare Konstruktion gegeben. Durch die Betätigung des Betätigungsmechanismus 8 kann gegen die Kraft der Vorbelastungseinrichtung 17 der in der Feststellposition 7 durch die Feststelleinrichtung 6 festgestellte Tragarm 3 in eine Drehposition überführt werden. In der Drehposition ist der Tragarm 3 um die Drehachse 4a des Achsteils 4 schwenkbar. Der Betätigungsmechanismus 8 umfasst hier insgesamt drei Übertragungselemente 11, welche alle als flexible Zugelemente 19 und genauer als Zugseile ausgeführt sind. Das erste Übertragungselement 11 ist hier mit der Feststelleinrichtung 6 verbunden. Das erste Übertragungselement 11 ist hier als Zugseil ausgeführt, welches durch den hohlen Abschnitt des Achsteils 10 axial unten aus dem Achsteil 4 herausgeführt und radial zum Tragteil 5 geführt und angeordnet ist. An dem ersten Übertragungselement 11 ist hier mittels einer Kupplungseinheit 206 ein weiteres Übertragungselement 11 verbunden, welches weiter radial entlang des Tragteils 5 nach außen geführt ist. Das zweite Zugelement 19 erstreckt sich horizontal entlang des Tragteils 5 bis zur Kraftrichtungseinheit 200 geführt. Das flexible Zugelement 19 ist an der Kraftrichtungseinheit 200 geführt und nach unten umgelenkt. Hier sind zwei Kraftrichtungseinheiten 200 angeordnet, um eine in das flexible Zugelement 19 eingeleitete Betätigungskraft direkt zur Betätigung der Feststelleinrichtung 6 zu leiten. An der mittleren Kraftrichtungseinheit 200 ist ein weitere mittels einer weiteren Kupplungseinheit 206 ein drittes Übertragungselement 11 verbunden. Für das dritte Übertragungselement 11 ist ebenfalls eine Kraftrichtungseinheit 200 vorgesehen.

Die Kraftrichtungseinheiten 200 umfassen hier jeweils ein Anschlagteil 201, welches fest mit dem Tragteil 5 des Tragarms 3 fest verbunden ist. An dem flexiblen Zugelement 19 ist in jeweils in der Nähe des Halteelements 201 ein Sperrteil 203 angeordnet und kraftschlüssig mit dem flexiblen Zugelement 19 verbunden. Zieht ein Benutzer an dem Betätigungselement 12, wird das Sperrteil an dem Anschlagteil 201 einer Kraftrichtungseinheit 200 bewegt, welche einen größeren radialen Abstand 34a zu dem Betätigungselement 12 aufweist. Dadurch wird die Kraftweiterleitung innerhalb des Übertragungselements 11 gezielt zu der Feststelleinrichtung 6 weitergeleitet. Das flexible Zugelement 19 kann nicht bei der Betätigung rückwärts dem führenden Anschlagteil 201 gezogen werden. Dadurch steht bevorzugt ein kurzer und definierter Betätigungsweg 19a zur Verfügung.

Ein Griffknopf des Betätigungselements 12 ist direkt benachbart zu der Aufnahmeschiene 23 angeordnet. So wird eine komfortable und einfache Betätigung ermöglicht. Ein Anschlagteil 201 ist auf etwa der Hälfte der Länge 34 des Tragteils 5 des Tragarms 3 radial außerhalb der Befestigungseinrichtung 2 angeordnet. Das zweite Anschlagteil 201 an einem radialen Abstand 34 angeordnet, welcher ungefähr 75 % der Länge 34 des Tragarms 3 der Radiologiehalteeinrichtung 1 entspricht. Dadurch wird eine komfortable Betätigung eines Benutzers in der Nähe der Halteeinrichtung 9 ermöglicht. Außerdem kann hier die Kraft zum Verschwenken bei mit einem großen radialen Abstand 34a zur Drehachse 4a, d. h. mit einem großen Hebelarm, eingeleitet werden.

Bei einer Betätigung der Feststelleinrichtung 6 durch das erste Betätigungselement 12, welches den kleineren radialen Abstand 34a zur Drehachse 4a aufweist, wird hier vorteilhaft durch die in einem größeren radialen Abstand 34a angeordnete Kraftrichtungseinheit 200 sichergestellt, dass das Zugelement nicht aus der Führung des Anschlagteils 201 herausgezogen wird. Das Sperrteil 203 sperrt eine Bewegung des flexiblen Zugelements 19. Die Betätigungskraft wird dadurch direkt zur Feststelleinrichtung 6 zur Betätigung geleitet. Die flexiblen Zugelemente 19 können durch die Kraftrichtungseinheiten 200 nicht ungewollt bzw. undefiniert durchhängen. Ein Betätigungsweg 19a ist hier durch die Kraftrichtungseinheit 200 definiert und vorgebbar. Im Wesentlichen entspricht der Betätigungsweg 19a hier dem zur Betätigung erforderlichen Hub eines Fixierelements 13, welches durch zur Überführung des Tragarms 3 in die Drehposition aus einer Vertiefung 4 der Feststelleinrichtung 6 gezogen wird.

Figur 2 zeigt eine Draufsicht auf die Radiologieanlage 100, umfassend ein Radiologiegerät 50 und einen Tragarm 3. Die Befestigungseinrichtung 2, vgl. Figur 1, ist nicht dargestellt, sodass die Feststelleinrichtung 6 in der Draufsicht sichtbar ist. Der Tragarm 3 ist um die Drehachse 4a des als Rohrteil ausgeführten Achsteils 4 schwenkbar.

Die Feststelleinrichtung 6 umfasst ein Fixierelement 13, welches an dem Tragarm 3 fixiert ist und durch die Vorbelastungseinrichtung 17 in die Feststellposition 7 vorbelastet wird. Das Fixierelement 13 ist als Bolzen ausgeführt, welcher in eine Vertiefung 14 eines Lochkreises 15 der Feststelleinrichtung 6 eingreift. Der Durchmesser der Vertiefungen 15 ist deutlich größer als der Durchmesser des Achsteils 16. So kann die Feststelleinrichtung 6 konstruktiv einfach radial außerhalb des Achsteils 4 angeordnet werden und erlaubt eine sichere Fixierung.

Es ist eine Begrenzungseinrichtung 25 für den Schwenkwinkel vorhanden. Die Begrenzungseinrichtung 25 für den Schwenkwinkel ist als mechanischer Anschlag ausgeführt, der den Schwenkwinkel des Tragarms 3 begrenzt. Der Anschlag ist in einer Vertiefung 14 befestigt.

Der Tragarm 3 ist durch eine Stoßeinrichtung 28 vor Stößen und gegen Beschädigungen geschützt. Gleichzeitig bildet die Stoßeinrichtung 28 einen wirksamen Schutz vor Beschädigung anderer Gegenstände.

An der Halteeinrichtung 9 ist eine Lagerungseinheit 31 für Patienten befestigt, welche durch die motorische betriebene Gurteinheit 32 auf und ab gefahren werden kann. Hier ist noch eine zweite Halteeinrichtung 9 an dem Tragteil 5 aufgenommen.

An dem Gehäuse der Gurteinheit 32 kann ein Betätigungsknopf 31a vorgesehen sein (vgl. Fig. 8), der zur Betätigung einer z. B. mit einer Wickelfeder angetriebenen (und hier nicht sichtbaren, da im Inneren angeordnete) Aufrolleinheit dient. Bei Betätigung des Knopfes 31a kann der Gurt (ohne Kraftbelastung) selbsttätig eingezogen und aufgewickelt werden oder aber der Gurt kann gegen die Kraft der Wickelfeder ausgezogen werden. Vorzugsweise steht eine Gurtlänge von wenigstens 200 oder 300 mm zur Verfügung. Die ausziehbare Gurtlänge kann in vorteilhaften Ausgestaltungen auch 500 mm oder 750 mm oder mehr betragen. Das hat in hohen Räumen den Vorteil, dass der Haltegriff der Halteeinrichtung 9 bei Nichtbenutzung so hoch gefahren werden kann, dass er bei Nichtbenutzung nicht stört und man nicht mit dem Kopf dagegen stoßen kann. Alternativ ist es auch möglich, den Haltegriff bei Nichtbenutzung an einen der Haken 5a zu hängen.

Gestrichelt eingezeichnet ist in Figur 2 eine Variante, bei der der Tragarm 3 an dem Radiologiegerät 50 aufgenommen oder daran ausgebildet ist. Das hat den Vorteil, dass die Befestigungseinrichtung 2 direkt an dem Radiologiegerät 50 aufgenommen ist, was eine insgesamt kompakte Struktur ermöglicht.

An den in Figuren 1 und 2 sichtbaren Haken 5a können Gegenstände aufgehangen werden. Beispielsweise können auch Infusionsbeutel daran befestigt oder Patientenunterlagen aufgehangen werden. Die Haken 5a können seitlich an dem Tragteil 5 befestigt werden, z. B. angeschweißt werden. Möglich ist es auch, dass die Haken 5a über Rohrschellen oder auch spezielle Befestigungssysteme ortsfest oder auch verschiebbar an dem Tragteil aufgenommen und befestigt werden.

Entlang des Tragteils 5 des Tragarms 3 sind hier die drei Kraftrichtungseinheiten 200 seitlich angeordnet. Die Anschlagteile 201 der Kraftrichtungseinheiten 200 umfassen und führen die flexiblen Zugelemente 19 horizontal entlang des Tragteils 5. Die Sperrteile 203 sind fest mit den flexiblen Zugelementen 19 verbunden. Die Betätigungselemente 12 sind hier nicht dargestellt. An den Haken 5a kann beispielsweise ein Infusionsbeutel 30 angehangen werden.

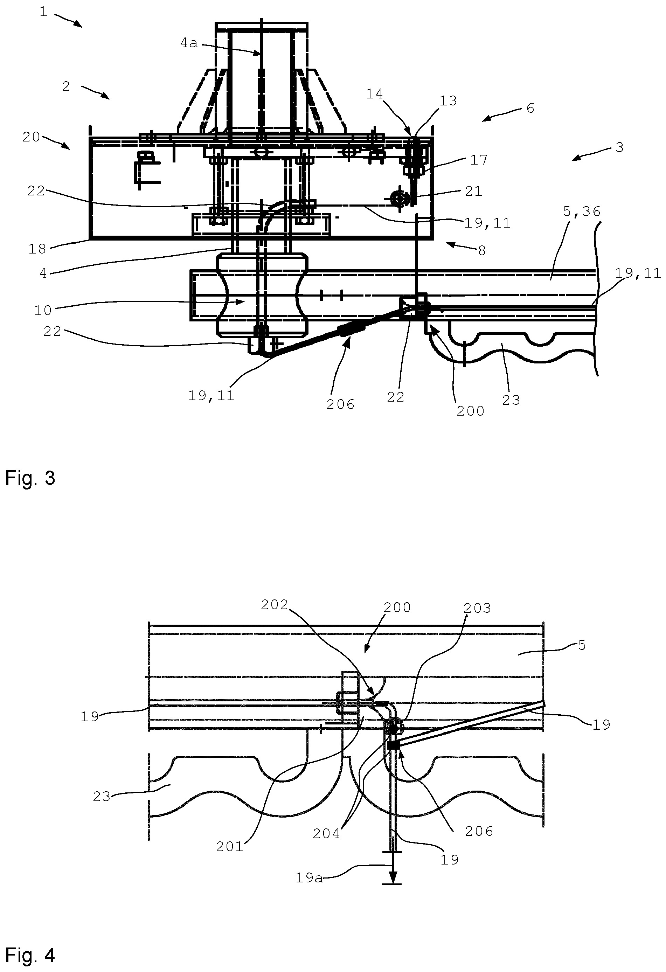

Figur 3 zeigt eine vergrößerte Schnittdarstellung eines Achsteils 4 und eines Teils des Tragteils 5 der Radiologiehalteeinrichtung 1, vgl. Bereich I in Figur 1. Die Befestigungseinrichtung 2 ist als Befestigungskonsole ausgeführt. Die Befestigungseinrichtung 2 ist an der Decke des nicht dargestellten Radiologieraums durch eine Verschraubung befestigbar. An der Befestigungseinrichtung 2 ist der schwenkbare Tragarm 3 aufgenommen und gelagert. Dazu ist das schwenkbare Achsteil 4 direkt mit der Befestigungseinrichtung 2 verbunden und daran angeordnet. Das Tragteil 5 erstreckt sich quer zum Achsteil 4. Der Tragarm 3 ist um die zentrale Drehachse 4a des Achsteils 4 verschwenkbar.

Das Achsteil 4 ist durch eine Zwischendecke durchgeführt. Die Zwischendecke ist nicht vollständig dargestellt.

Eine Blendeneinrichtung 18 ist mittig an dem Achsteil 4 angeordnet. Dadurch wird die Öffnung in der Zwischendecke verschlossen und abgedeckt. Eine magnetische Schirmung, welche sich in oder an der Zwischendecke befindet, kann dadurch effektiv abgedeckt und verschlossen werden. Die Blendeneinrichtung 18 umfasst eine magnetische Schirmung, sodass die verbleibende Öffnung der magnetischen Schirmung möglichst klein ist.

Die Feststelleinrichtung 6 stellt den Tragarm 3 durch ein Fixierelement 13 in der dargestellten Feststellposition 7 fest. Regelmäßig wird dieser durch eine Vorbelastungseinrichtung 17, welche hier als Spiralfeder ausgebildet ist, in die Feststellposition 7 vorbelastet. Der Tragarm 3 ist verdrehsicher in der Feststellposition 7 fixiert. So kann eine sichere Nutzung der Radiologiehalteeinrichtung 1 gewährleistet werden.

Durch den Betätigungsmechanismus 8 ist die Feststelleinrichtung 6 betätigbar. Das Fixierelement 13 ist mit dem Übertragungselement 11 verbunden. Das Übertragungselement 11 ist als flexibles Zugelement 19, genauer als Zugseil, ausgeführt.

Das Übertragungselement 11 wird durch eine Führungsrolle 21 radial auf das Achsteil 4 gelenkt. Das Übertragungselement 11 wird durch die Führungsrolle 21 umgelenkt und durch eine Führungseinheit 20 innerhalb des Achsteils 4 entlang der Achse des Achsteils 4 durch die Führungseinheit 20 geführt. Die Führungseinheit 20 umfasst hier ein Rohr. Zur Umlenkung des Übertragungselements 11 ist das Rohr gebogen.

Am unteren Ende des Achsteils 4 wird das Übertragungselement 11 axial aus dem Achsteil 4 herausgeführt. Am unteren Ende des Achsteils 4 ist eine Umlenkhülse 22 angebracht. Die Umlenkhülse 22 ist als konisches Rohrteil oder Hülse ausgeführt. Die Umlenkhülse 22, ist vorzugsweise aus einem Kunststoff gefertigt, welcher eine besonders gleitfähige und glatte Oberfläche aufweist und so einen geringen Reibungswiderstand bietet. Dadurch kann das Übertragungselement 11 auf einem kleinen Radius in Richtung des Tragteils 5 des Tragarms 3 umgelenkt werden. Das Tragteil 5 kann ein separates Teil sein oder einstückig mit dem Tragarm 3 ausgebildet sein.

Das Übertragungselement 11 ist innerhalb des Achsteils 4 durch die Zwischendecke durchgeführt. So muss es nicht separat außerhalb des Achsteils 4 durch die Zwischendecke geführt werden. Eine Öffnung der Zwischendecke ist deshalb deutlich kleiner, als wenn das Zugseil einfach gerade und parallel zur Achse des Achsteils 4 herunterhängen würde. Die notwendige Öffnung der magnetischen Schirmung in einer Zwischendecke ist deutlich kleiner und vorteilhafter verschließbar als bei den im Stand der Technik bekannten Ausführungen.

Am Tragteil 5 wird das Übertragungselement 11 in horizontale Richtung entlang der Achse des Tragteils 5 umgelenkt und weiter entlang der Achse des Tragteils 5 geführt. Ein zweites Übertragungselement 11 ist hier durch die Kupplungseinheit 206 mit dem ersten Übertragungselement verbunden. Durch die Kraftrichtungseinheit 200 wird hier eine optimale Weiterleitung der eingeleiteten Betätigungskraft sichergestellt. Ein undefiniertes und vor allem nicht beabsichtigtes Durchhängen des flexiblen Zugelements 19 wird vermieden.

In Figur 4 ist eine vergrößerte Darstellung eines Bereichs des Tragarms 3 mit einer Kraftrichtungseinheit 200 im betätigten Zustand, vgl. Bereich II in Figur 1. Das Anschlagteil 201 der Kraftrichtungseinheit 200 fest mit dem Tragteil 5 des Tragarms verbunden, zum Beispiel angeschraubt oder verschweißt, und schließt und führt das flexible Zugelement 19 hülsenförmig.

Außerdem wird das flexible Zugelement 19 durch das Anschlagteil 201 geführt. An dem flexiblen Zugelement 19 ist das Sperrteil 203 kraftschlüssig aufgenommen. Die Position des Sperrteils 203 an dem flexiblen Zugelement 19 ist variabel einstellbar.

Ein Konturverlauf 202 des Anschlagteils 201 erweitert sich mit steigendem Abstand 34a von der Drehachse 4a. Dadurch kann das flexible Zugelement 19 vorteilhaft an dem Anschlagteil 201 so umgelenkt werden, dass eine Betätigung der Feststelleinrichtung 6 möglich ist. Das Zugelement 19 ist nach unten umgelenkt, wo das nicht dargestellte Betätigungselement 12 angeordnet ist.

Das Sperrteil 203 ist kugelförmig und innen hohl ausgebildet. Das Zugelement 19 ist durch das Sperrteil 203 durchgeführt. An dem Zugelement 19 ist ein Knotenabschnitt 204 ausgebildet, welcher innen an dem Sperrteil 203 aufgenommen ist. Das Sperrteil 203 umfasst hier außerdem eine als Schraubeneinheit 205 ausgeführte Aufnahmeeinheit 205, welche als Madenschraube ausgeführt ist. Das Zugelement 19 ist durch die Madenschraube durchgeführt. Die Madenschraube ist in das Sperrteil 203 eingeschraubt. So ist das Sperrteil 203 hier mit dem Zugelement 19 kraftschlüssig verbunden.

Das kugelförmige Sperrteil 203 ist an dem Anschlagteil 201 der Kraftrichtungseinheit 200 aufnehmbar. Das Sperrteil 203 zentriert sich hier an dem rotationssymmetrischen hülsenförmigen Anschlagteil 201. An dem Zugelement 19 ist mittels einer Kupplungseinheit 206 ein weiteres Zugelement 19 kraftschlüssig verbunden. Die Kupplungseinheit wird hier durch einen Knotenabschnitt 204 gebildet. Das weitere Zugelement 19 ist an dem ersten Zugelement 19 durch einen Knotenabschnitt 204 kraftschlüssig angeordnet.

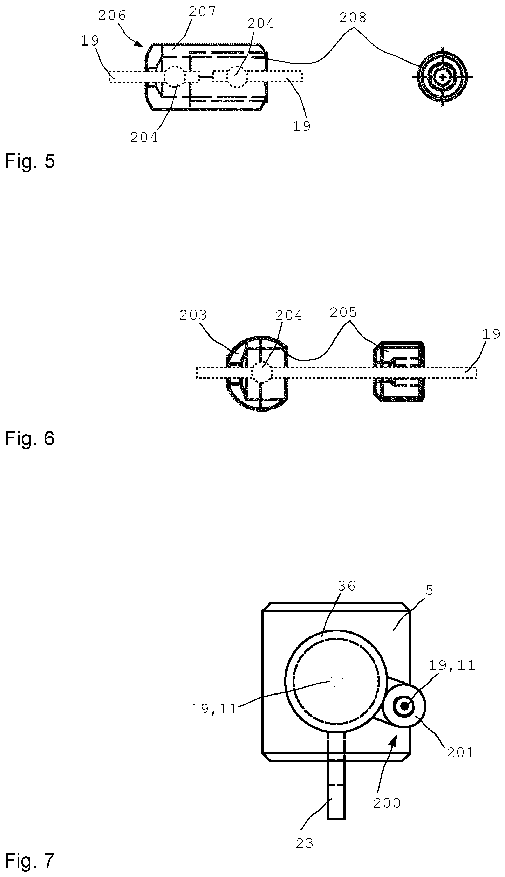

Figur 5 zeigt eine Ausführungsform der Kupplungseinheit 206 in einer Schnittansicht. Die Kupplungseinheit 206 umfasst ein Hülsenteil 207. Innerhalb des Hülsenteils sind zwei Übertragungselemente 11 angeordnet. An den hier als flexible Zugelemente 19 ausgeführten Übertragungselementen 11 ist je ein Knotenabschnitt 204 ausgebildet. Das Hülsenteil 207 der Kupplungseinheit 206 wird durch eine Schraubeneinheit 208 verschlossen. Die Schraubeneinheit 208 ist hier als Madenschraube ausgeführt, durch welche das eine Zugelement 19 durchgeführt ist. Im verschlossenen Zustand sind die beiden Zugelemente 19 kraftschlüssig verbunden. Die Darstellung des Zugelements 19 durch gestrichelte Linien soll hier eine mögliche Anordnung des flexiblen Zugelements 19 an der Kupplungseinheit 206 verdeutlichen. Darüber hinaus sind weitere Anordnungen und Verbindungen der Zugelemente 19 möglich und denkbar. Dies gilt auch für die nachfolgenden Figuren.

Figur 6 zeigt eine Schnittansicht eines Sperrteils 203 der Kraftrichtungseinheit 200. Das Sperrteil 203 ist kugelförmig als Griff ausgebildet. Das Sperrteil 203 ist kugelförmiges Hülsenteil 207 ausgebildet. Das Zugelement 19 ist durch einen daran ausgebildeten Knotenabschnitt 204 an dem Sperrteil 203 aufgenommen. Das Sperrteil 203 wird durch die Aufnahmeeinheit 205, welche hier als Schraubeneinheit 205 ausgebildet ist, verschlossen, sodass eine kraftschlüssige Verbindung vorliegt. Das Sperrteil 203 kann hier an nahezu jeder beliebigen Position des flexiblen Zugelements 19 angeordnet werden.

Figur 7 zeigt eine Schnittansicht durch das Tragteil 5 des Tragarms 3 der Radiologiehalteeinrichtung 1 quer zur Achse des Tragteils 5, vgl. Bereich III in Figur 1. Das Anschlagteil 201 ist seitlich an dem Tragteil 5 angeordnet. Das Anschlagteil 201 umfasst und führt das flexible Zugelement 19. Das Sperrteil 203 ist nicht dargestellt. Es ist auch möglich, dass das Zugelement 19 durch einen hohlen Abschnitt eines Rohrelements 36 des Tragteils 5 geführt wird (siehe gestrichelte Linie). So ist das Übertragungselement 11 vorteilhaft vor einer Verschmutzung geschützt. Ein Benutzer kann sich nicht an dem Zugelement 19 verfangen. Das Verletzungsrisiko ist minimiert.

Figur 8 zeigt eine perspektivische Ansicht einer Halteeinrichtung 9 für eine erfindungsgemäße Radiologiehalteeinrichtung. Die Halteeinrichtung 9 umfasst hier ein Hakenelement 29, mit welchem die Halteeinrichtung in einem Einhängepunkt 24 der Aufnahmeschiene 23 aufnehmbar ist. An der Halteeinrichtung 9 ist ein weiterer Lagerungshaken 29a zur Aufnahme von zusätzlichen Gegenständen, wie zum Beispiel einem Infusionsbeutel 30, angeordnet. Vorteilhaft kann der Haltegriff 31 hochgeklappt und an dem Lagerungshaken 29a eingehängt werden. Ein Risiko für Verletzungen und Stöße wird dadurch minimiert. Durch die langen Hakenschenkel sind große Haltewinkel 35 von bis zu 60° und mehr möglich, ohne das die Gefahr besteht, dass der Hakenschenkel aus der Aufnahmeschiene 23 herausrutscht.

In Figur 8 ist der Knopf 31a zu erkennen, der Gurteinheit 32 und hier an dem Haltegriff der Halteeinrichtung 9 ausgebildet ist, um den Gurt 32e über eine nicht sichtbare und im Inneren angeordnete Wickelfeder aufzuwickeln oder gegen die Spannkraft der Wickelfeder auszuziehen. Der Haltegriff ist mit der Gurteinheit 32 drehbar an der Stange 32b aufgenommen. Die Gurteinheit 32 ist über das Gleitelement 32d verschwenkbar aufgenommen. Das Gleitelement 32d dient als Lagereinheit und ist hier zwischen dem oberen Bügel des Halters 32a und dem vergrößerten Kopf 32d der Stange 32b aufgenommen, um eine leichtgängige Verschwenkung der Gurteinheit in Richtung eines Patienten zu ermöglichen. Grundsätzlich ist zwar auch der Gurt 32e in sich tordierbar, aber das kann im Betrieb zu Problemen führen, wenn sich der Gurt schief und nicht ordnungsgemäß aufwickelt.

### Bezugszeichenliste:

| | | | |
|---|---|---|---|
| 1 | Radiologiehalteeinrichtung | 29 | Hakenelement |
| 2 | Befestigungseinrichtung | 29a | Lagerungshaken |
| 3 | Tragarm | 30 | Infusionsbeutel |
| 4 | Achsteil von 3 | 31 | Lagerungseinheit |
| 4a | Drehachse | 31a | Knopf |
| 5 | Tragteil von 3 | 32 | Gurteinheit |
| 5a | Haken | 32a | Halter |
| 6 | Feststelleinrichtung | 32b | Stange |
| 7 | Feststellposition | 32c | Gleitelement |
| 8 | Betätigungsmechanismus | 32d | Lagerung |
| 9 | Halteeinrichtung | 32e | Gurt |
| 10 | hohler Abschnitt des Achsteils 4 | 34 | Länge von 3 |
| | | 34a | radialer Abstand zu 4 |
| 11 | Übertragungselement von 8 | 35 | Haltewinkel von 9 |
| 12 | Betätigungselement von 8 | 36 | Rohrelement von 5 |
| 13 | Fixierelement von 6 | 50 | Radiologiegerät |
| 14 | Vertiefung von 6 | 51 | Röntgengerät |
| 15 | Durchmesser des Lochkreises | 52 | MRT-Gerät |
| 16 | Durchmesser des Achsteils 4 | 53 | CT-Gerät |
| 17 | Vorbelastungseinrichtung | 54 | Strahlentherapiegerät |
| 18 | Blendeneinrichtung | 100 | Radiologieanlage |
| 19 | flexibles Zugelement von 11 | 200 | Kraftrichtungseinheit |
| 19a | Betätigungsweg von 19 | 201 | Anschlagteil von 200 |
| 20 | Führungseinheit | 202 | Konturverlauf von 201 |
| 21 | Führungsrolle | 203 | Sperrteil |
| 22 | Umlenkhülse | 204 | Schlaufenabschnitt, |
| 23 | Aufnahmeschiene | Kno | tenabschnitt von 19 |
| 24 | Einhängepunkt | 205 | Schraubeneinheit, Aufnahmeeinheit von 203 |
| 25 | Begrenzungseinrichtung für den Schwenkwinkel | | |
| | | 206 | Kupplungseinheit |
| 26 | Arbeitstisch für Patienten | 207 | Hülsenteil von 206 u. 203 |
| 27 | Schutzhülse | 208 | Festsetzeinheit, Schraubeneinheit |
| 28 | Stoßschutzeinrichtung | | |

## Patentansprüche

1. Radiologiehalteeinrichtung (1), insbesondere für ein Radiologiegerät (50) wie ein Röntgengerät (51), MRT-Gerät (52), CT-Gerät (53) oder Strahlentherapiegerät (54), umfassend eine Befestigungseinrichtung (2) und wenigstens einen daran schwenkbar aufgenommenen Tragarm (3), mit wenigstens einem Achsteil (4) und wenigstens einem Tragteil (5),
wobei das Achsteil (4) des Tragarms (3) schwenkbar an der Befestigungseinrichtung (2) aufgenommen ist, um den Tragarm (3) zu verschwenken,
und eine Feststelleinrichtung (6), um den Tragarm (3) in wenigstens einer Feststellposition (7) festzustellen, und einen Betätigungsmechanismus (8) mit wenigstens einem Übertragungselement (11) und wenigstens einem Betätigungselement (12), um die Feststelleinrichtung (6) zu betätigen,
und wenigstens eine an dem Tragarm (3) befestigbare Halteeinrichtung (9), um z. B. einem Patienten eine Haltemöglichkeit z. B. beim Aufrichten oder Umlagern zur Verfügung zu stellen,
**dadurch gekennzeichnet, dass** das Übertragungselement (11) wenigstens abschnittsweise als flexibles Zugelement (19) ausgebildet ist,
dass der Betätigungsmechanismus (8) wenigstens eine Kraftrichtungseinheit (200) aufweist, die eine Kraftweiterleitung in eine Richtung sperrt, um eine in das flexible Zugelement (19) eingeleitete Betätigungskraft gezielt zur Betätigung zu der Feststelleinrichtung (6) zu leiten
und dass die Kraftrichtungseinheit (200) wenigstens ein Anschlagteil (201) umfasst, welches an dem Tragarm (3) angeordnet ist.

2. Radiologiehalteeinrichtung nach Anspruch 1, wobei der Betätigungsmechanismus (8) durch wenigstens zwei Betätigungselemente (12) unabhängig voneinander betätigbar ist.

3. Radiologiehalteeinrichtung (1) nach einem der vorhergehenden Ansprüche, wobei der Betätigungsmechanismus (8) wenigstens zwei oder mehr Kraftrichtungseinheiten (200) umfasst.

4. Radiologiehalteeinrichtung (1) nach einem der vorhergehenden Ansprüche, wobei ein Betätigungsweg (19a) des Zugelements (19) zur Betätigung der Feststelleinrichtung (6) kleiner ist als ein doppelter Durchmesser des Betätigungselements (11) und/oder wobei ein Betätigungsweg (19a) im Wesentlichen einem Hub zur Betätigung der Feststelleinrichtung (6) entspricht.

5. Radiologiehalteeinrichtung (1) nach einem der vorhergehenden Ansprüche, wobei das Zugelement (19) an dem Anschlagteil (201) aufgenommen ist.

6. Radiologiehalteeinrichtung (1) nach dem vorhergehenden Anspruch, wobei sich eine Kontur (202) des Anschlagteils (201) mit steigendem radialen Abstand (4a) von der Schwenkachse (34) aufweitet.

7. Radiologiehalteeinrichtung (1) nach einem der beiden vorhergehenden Ansprüche, wobei der radiale Abstand (34a) zur Schwenkachse (4a) wenigstens 20 % einer maximalen radialen Länge (34) des Tragarms (3) entspricht.

8. Radiologiehalteeinrichtung (1) nach einem der vorhergehenden Ansprüche, wobei die Kraftrichtungseinheit (200) wenigstens ein Sperrteil (203) aufweist, welches an dem flexiblen Zugelement (19) aufgenommen ist,
wobei das Sperrteil (202) an dem Zugelement (19) befestigt ist oder wobei eine Bewegbarkeit des Sperrteils (202) gegenüber dem Zugelement (19) in wenigstens eine Richtung begrenzt ist,
und wobei das Sperrteil (203) an dem Anschlagteil (201) abstützbar ist.

9. Radiologiehalteeinrichtung (1) nach einem der vorhergehenden Ansprüche, wobei der Betätigungsmechanismus (8) wenigstens zwei Übertragungselemente (11) umfasst, welche durch wenigstens eine Kupplungseinheit (206) kraftschlüssig miteinander verbunden sind,
wobei die Kupplungseinheit (206) wenigstens ein Hülsenteil (207) und wenigstens eine Schraubeneinheit (208) umfasst, an welchen die Übertragungselemente (11) und insbesondere die flexiblen Zugelemente (19) angeordnet sind.

10. Radiologiehalteeinrichtung (1) nach einem der vorhergehenden Ansprüche, wobei das Übertragungselement (11) durch wenigstens einen hohlen Abschnitt (10) des Achsteils (4) durchgeführt ist.

11. Radiologiehalteeinrichtung (1) nach einem der vorhergehenden Ansprüche, wobei die Feststelleinrichtung (6) wenigstens ein Fixierelement (13) umfasst und wobei das Fixierelement (13) von einer Feststellposition (7) in eine Drehposition überführbar ist, in der eine Winkelstellung des Tragarms (3) gegenüber der Befestigungseinrichtung (2) veränderbar ist, wobei bei einer Betätigung eines Betätigungselements (12) das Anschlagteil (201), welches in einem größeren radialen Abstand (34a) angeordnet ist als das betätigte Betätigungselement (12), eine Bewegung des als Zugelement (19) ausgebildeten Übertragungselements (12) mittels des Sperrteils (203) sperrt, sodass die in das Zugelement (19) eingeleitete Betätigungskraft im Wesentlichen auf das Fixierelement (13) der Feststelleinrichtung (6) wirkt und wobei wenigstens eine Vorbelastungseinrichtung (17) umfasst ist, durch welche das Fixierelement (13) in die Feststellposition (6) vorbelastbar ist,
und wobei wenigstens eine Vorbelastungseinrichtung (17) umfasst ist, durch welche das Fixierelement (13) in die Feststellposition (6) vorbelastbar ist.

12. Radiologiehalteeinrichtung (1) nach einem der vorhergehenden Ansprüche, wobei wenigstens eine Begrenzungseinrichtung (25) zur Begrenzung wenigstens eines Schwenkwinkels umfasst ist.

13. Radiologiehalteeinrichtung (1) nach einem der vorhergehenden Ansprüche, wobei wenigstens eine Aufnahmeschiene (23) mit wenigstens einem Einhängepunkt (24) entlang des Tragarms (3) ausgebildet ist und wobei sich die Aufnahmeschiene (23) über einen wesentlichen Teil der radialen Länge (34) des Tragarms (3) erstreckt,
wobei wenigstens eine Halteeinrichtung (9) in wenigstens einen Einhängepunkt (24) der Aufnahmeschiene (23) einhängbar ist und wobei insbesondere an der Aufnahmeschiene (23) eine Mehrzahl miteinander verbundener Einhängepunkte (24) ausgebildet ist
und wobei die Halteeinrichtung (9) wenigstens ein Hakenelement (29) zum Einhängen an wenigstens einem Einhängepunkt (24) umfasst und/oder wobei die Halteeinrichtung (9) wenigstens eine Gurteinheit (32), mit welcher die Länge der Halteeinrichtung (9) variabel einstellbar ist und/oder wobei die Gurteinheit (32) im gebrauchsfertigen Zustand wenigstens um eine quer zur Horizontalen ausgerichtete Achse drehbar gelagert ist.

14. Radiologiehalteeinrichtung (1) nach einem der beiden Ansprüche 5 und 6, wobei wenigstens ein Anschlagteil (201) in einem radialen Abstand (34a) zur Schwenkachse (4a) angeordnet ist, welcher wenigstens 20 % einer maximalen radialen Länge (34) des Tragarms (3) entspricht und/oder wobei wenigstens ein Anschlagteil (201) radial außerhalb der Befestigungseinrichtung (2) angeordnet ist.

15. Radiologieanlage (100), umfassend wenigstens ein Radiologiegerät (50) und wenigstens eine Radiologiehalteeinrichtung (1) nach wenigstens einem der vorhergehenden Ansprüche.

## Claims

1. Radiology holding device (1), in particular for a radiology apparatus (50) such as an X-ray apparatus (51), MRT apparatus (52), CT apparatus (53) or radiotherapy apparatus (54), comprising an attachment device (2) and pivotally accommodated thereat, at least one supporting arm (3) with at least one axle component (4) and at least one supporting component (5),
wherein the axle component (4) of the supporting arm (3) is pivotally accommodated on the attachment device (2) for pivoting the supporting arm (3),
and a locking device (6) for locking the supporting arm (3) in at least one locking position (7), and an actuating mechanism (8) including at least one transfer component (11) and at least one actuating member (12) for actuating the locking device (6), and at least one holding device (9) provided for attachment to the supporting arm (3), e.g. to provide a patient with a holding means e.g. for sitting up or shifting,
**characterized in**
**that** the transfer component (11) is, at least in sections, configured as a flexible pull member (19),
**that** the actuating mechanism (8) comprises at least one force direction unit (200) which locks force transfer in one direction, so as to specifically transfer to the locking device (6), an operating force introduced into the flexible pull member (19) for actuating the locking device,
and **that** the force direction unit (200) comprises at least one stopper part (201) disposed on the supporting arm (3).

2. The radiology holding device according to claim 1, wherein the actuating mechanism (8) can be actuated by at least two actuating members (12) independently of one another.

3. The radiology holding device (1) according to any of the preceding claims, wherein the actuating mechanism (8) comprises at least two or more force direction units (200).

4. The radiology holding device (1) according to any of the preceding claims, wherein an actuation travel (19a) of the pull member (19) for actuating the locking device (6) is less than twice the diameter of the actuating member (11), and/or wherein an actuation travel (19a) substantially corresponds to a stroke length for actuating the locking device (6).

5. The radiology holding device (1) according to any of the preceding claims, wherein the pull member (19) is accommodated on the stopper part (201).

6. The radiology holding device (1) according to the preceding claim, wherein the contour (202) of the stopper parts (201) expands with increasing radial distance (4a) from the pivot axis (34).

7. The radiology holding device (1) according to any of the two preceding claims, wherein the radial distance (34a) from the pivot axis (4a) corresponds to at least 20 % of the maximal radial length (34) of the supporting arm (3).

8. The radiology holding device (1) according to any of the preceding claims, wherein the force direction unit (200) comprises at least one blocking part (203) which is accommodated on the flexible pull member (19),
wherein the blocking part (202) is fixed to the pull member (19), or wherein movability of the blocking part (202) relative to the pull member (19) is limited in at least one direction, and wherein the blocking part (203) can be propped on the stopper part (201).

9. The radiology holding device (1) according to any of the preceding claims, wherein the actuating mechanism (8) comprises at least two transfer components (11), which are interconnected in a force-fit by at least one coupling unit (206),
wherein the coupling unit (206) comprises at least one sleeve part (207) and at least one screw unit (208), where the transfer components (11) and in particular the flexible pull members (19) are disposed.

10. The radiology holding device (1) according to any of the preceding claims, wherein the transfer component (11) is passed through at least one hollow section (10) of the axle component (4).

11. The radiology holding device (1) according to any of the preceding claims, wherein the locking device (6) comprises at least one fixing member (13), and wherein the fixing member (13) can be transferred from a locking position (7) to a rotary position, in which the angular position of the supporting arm (3) relative to the attachment device (2) can be changed,
wherein with actuation of an actuating member (12), the stopper part (201) which is disposed at a larger radial distance (34a) than is the actuated actuating member (12), locks movement of the transfer component (12) configured as a pull member (19) by means of the blocking part (203), so that the operating force introduced into the pull member (19) substantially acts on the fixing member (13) of the locking device (6),
and wherein at least one biasing device (17) is comprised, by means of which the fixing member (13) can be biased in the locking position (6).

12. The radiology holding device (1) according to any of the preceding claims, wherein at least one limiting device (25) for limiting at least one rotational angle is comprised.

13. The radiology holding device (1) according to any of the preceding claims, wherein at least one receiving rail (23) is configured with at least one hooking point (24) along the supporting arm (3), and wherein the receiving rail (23) extends over at least a substantial part of the radial length (34) of the supporting arm (3),
wherein at least one holding device (9) can be hooked into at least one hooking point (24) of the receiving rail (23), and
wherein a plurality of interconnected hooking points (23) are configured in particular on the receiving rail (23),
and wherein die holding device (9) comprises at least one hook member (29) for hooking in at least one hooking point (24),
and/or wherein the holding device (9) comprises at least one belt unit (32), with which the length of the holding device (9) is adjustable variably, and/or wherein the belt unit (32), when ready to use, is supported for rotation about at least one axis oriented transverse to the horizontal.

14. The radiology holding device (1) according to any of the two claims 5 and 6, wherein at least one stopper part (201) is disposed at a radial distance (34a) from the pivot axis (4a), corresponding at least to 20 % of the maximal radial length (34) of the supporting arm (3), and/or wherein at least one stopper part (201) is disposed radially outwardly of the attachment device (2).

15. Radiology system (100), comprising at least one radiology apparatus (50) and at least one radiology holding device (1) according to at least one of the preceding claims.

## Revendications

1. Dispositif de retenue utilisé en radiologie (1), notamment pour un appareil de radiologie (50) tel qu'un appareil à rayons X (51), un appareil IRM (52), un appareil de tomographie (53) ou un appareil de radiothérapie (54), comprenant un dispositif de fixation (2) et au moins un bras porteur (3) qui y est monté de manière articulable, avec au moins une pièce d'axe (4) et au moins une pièce porteuse (5),
la pièce d'axe (4) du bras porteur (3) étant montée articulable sur le dispositif de fixation (2) pour faire pivoter ledit bras porteur (3),
et un dispositif de verrouillage (6) pour verrouiller le bras porteur (3) dans au moins une position de verrouillage (7), ainsi qu'un mécanisme actionneur (8) doté d'au moins un élément de transmission (11) et d'au moins un élément actionneur (12) pour actionner le dispositif de verrouillage (6),
et au moins un dispositif de retenue (9) qui peut être fixé sur le bras porteur (3), par exemple pour offrir au patient une possibilité de s'accrocher, par exemple pour se redresser ou changer de position,
**caractérisé en ce**
**que** l'élément de transmission (11) est conçu au moins partiellement sous forme d'élément de traction flexible (19),
**que** le mécanisme actionneur (8) présente au moins une unité de direction de force (200) qui bloque une transmission de la force dans un sens afin de diriger de manière ciblée une force d'actionnement appliquée à l'élément de traction flexible (19) vers le dispositif de verrouillage (6) en vue de son actionnement,
et **que** l'unité de direction de force (200) comprend au moins une pièce de butée (201) qui est agencée sur le bras porteur (3).

2. Dispositif de retenue utilisé en radiologie selon la revendication 1, le mécanisme actionneur (8) pouvant être actionné par au moins deux éléments actionneurs (12) indépendamment l'un de l'autre.

3. Dispositif de retenue utilisé en radiologie (1) selon l'une quelconque des revendications précédentes, le mécanisme actionneur (8) comprenant au moins deux ou plusieurs unités de direction de force (200).

4. Dispositif de retenue utilisé en radiologie (1) selon l'une quelconque des revendications précédentes, un trajet d'actionnement (19a) de l'élément de traction (19) pour actionner le dispositif de verrouillage (6) étant inférieur au double du diamètre de l'élément actionneur (11), et/ou un trajet d'actionnement (19a) correspondant sensiblement à une course permettant d'actionner le dispositif de verrouillage (6).

5. Dispositif de retenue utilisé en radiologie (1) selon l'une quelconque des revendications précédentes, l'élément de traction (19) étant monté sur la pièce de butée (201).

6. Dispositif de retenue utilisé en radiologie (1) selon la revendication précédente, un contour (202) de la pièce de butée (201) s'élargissant au fur et à mesure qu'augmente la distance radiale (4a) par rapport à l'axe de pivot (34).

7. Dispositif de retenue utilisé en radiologie (1) selon l'une quelconque des deux revendications précédentes, la distance radiale (34a) par rapport à l'axe de pivot (4a) étant égale à au moins 20 % d'une longueur radiale maximale (34) du bras porteur (3).

8. Dispositif de retenue utilisé en radiologie (1) selon l'une quelconque des revendications précédentes, l'unité de direction de force (200) présentant au moins une pièce de blocage (203) qui est montée sur l'élément de traction flexible (19),
ladite pièce de blocage (202) étant fixée à l'élément de traction (19) ou une mobilité de la pièce de blocage (202) étant, par rapport à l'élément de traction (19), limitée dans au moins une direction,
et ladite pièce de blocage (203) pouvant venir s'appuyer sur la pièce de butée (201).

9. Dispositif de retenue utilisé en radiologie (1) selon l'une quelconque des revendications précédentes, le mécanisme actionneur (8) comprenant au moins deux éléments de transmission (11), qui sont reliés entre eux par adhérence au moyen d'au moins une unité d'accouplement (206), l'unité d'accouplement (206) comprenant au moins un élément de douille (207) et au moins un ensemble de vis (208) sur lesquels sont agencés les éléments de transmission (11) et notamment les éléments de traction flexibles (19).

10. Dispositif de retenue utilisé en radiologie (1) selon l'une quelconque des revendications précédentes, l'élément de transmission (11) passant au travers d'au moins une section creuse (10) de la pièce d'axe (4).

11. Dispositif de retenue utilisé en radiologie (1) selon l'une quelconque des revendications précédentes, le dispositif de verrouillage (6) comprenant au moins un élément de fixation (13), et ledit élément de fixation (13) pouvant être transféré d'une position de verrouillage (7) dans une position de pivotement à laquelle une position d'angle du bras porteur (3) peut être modifiée par rapport au dispositif de verrouillage (2),
au moment où un élément actionneur (12) est actionné, la pièce de butée (201) qui est agencée à une distance radiale (34a) supérieure à celle de l'élément actionneur (12) actionné, bloquant, au moyen de la pièce de blocage (203), un mouvement de l'élément de transmission (12) conçu sous la forme d'un élément de traction (19), de sorte que la force d'actionnement appliquée sur l'élément de traction (19) agit sensiblement sur l'élément de fixation (13) du dispositif de verrouillage (6),
et au moins un dispositif de précontrainte (17) étant compris, lequel permet d'appliquer une précontrainte sur l'élément de fixation (13) dans la position de verrouillage (6),
et au moins un dispositif de précontrainte (17) étant compris, lequel permet d'appliquer une précontrainte sur l'élément de fixation (13) dans la position de verrouillage (6).

12. Dispositif de retenue utilisé en radiologie (1) selon l'une quelconque des revendications précédentes, au moins un dispositif de délimitation (25) étant compris pour délimiter au moins un angle de pivotement.

13. Dispositif de retenue utilisé en radiologie (1) selon l'une quelconque des revendications précédentes, au moins un rail de montage (23) étant conçu doté d'au moins un point d'attache (24) le long du bras porteur (3), et ledit rail de montage (23) s'étendant sur une majeure partie de la longueur radiale (34) du bras porteur (3),
au moins un dispositif de retenue (9) pouvant être accroché à au moins un point d'attache (24) du rail de montage (23), et une pluralité de points d'attache (24) reliés entre eux étant réalisés notamment sur ledit rail de montage (23),
et le dispositif de retenue (9) comprenant au moins un élément crochet (29) pour accrocher à au moins un point d'attache (24), et/ou le dispositif de retenue (9) au moins un ensemble de sangles (32) avec lequel la longueur du dispositif de retenue (9) peut être ajustée de manière variable, et/ou l'ensemble de sangles (32), quand il prêt à l'emploi, est disposé mobile en rotation au moins autour d'un axe perpendiculaire à l'axe horizontal.

14. Dispositif de retenue utilisé en radiologie (1) selon l'une quelconque des deux revendications 5 et 6, au moins une pièce de butée (201) étant agencée à une distance radiale (34a) par rapport à l'axe de pivot (4a), ladite distance correspondant au moins à 20 % d'une longueur radiale maximale (34) du bras porteur (3), et/ou au moins une pièce de butée (201) étant agencée de manière radiale à l'extérieur du dispositif de fixation (2).

15. Installation de radiologie (100), comprenant au moins un appareil de radiologie (50) et au moins un dispositif de retenue utilisé en radiologie (1) selon au moins l'une quelconque des revendications précédentes.
